# EUROPÄISCHE PATENTSCHRIFT

(11) **EP 2 313 397 B1**
(45) Veröffentlichungstag und Bekanntmachung des Hinweises auf die Patenterteilung: **20.04.2016**
(21) Anmeldenummer: 09780993.3
(22) Anmeldetag: 23.07.2009
(51) Int. Cl.: C07D 403/12, A61K 31/519, A61P 35/00

(54) **CYCLOHEXYLOXY-SUBSTITUIERTE HETEROCYCLEN, DIESE VERBINDUNGEN ENTHALTENDE ARZNEIMITTEL, DEREN VERWENDUNG UND VERFAHREN ZU IHRER HERSTELLUNG**
CYCLOHEXYLOXY SUBSTITUTED HETEROCYCLES, MEDICINE CONTAINING THESE CONNECTIONS, THEIR APPLICATION AND PRODUCTION METHOD
HÉTÉROCYCLES SUBSTITUÉS PAR CYCLOHEXYLOXY, MÉDICAMENT CONTENANT CES LIAISONS, LEUR UTILISATION ET LEUR PROCÉDÉ DE FABRICATION

(30) Priorität: 08.08.2008 EP 08104995
(43) Veröffentlichungstag der Anmeldung: 27.04.2011
(73) Patentinhaber: Boehringer Ingelheim International GmbH, 55216 Ingelheim am Rhein (DE)
(72) Erfinder: HIMMELSBACH, Frank, 55216 Ingelheim Am Rhein (DE); JUNG, Birgit, 55216 Ingelheim Am Rhein (DE); LOTZ, Ralf, 55216 Ingelheim Am Rhein (DE)
(74) Vertreter: Simon, Elke Anna Maria
(86) Internationale Anmeldenummer: PCT/EP2009/059510
(87) Internationale Veröffentlichungsnummer: WO 2010/015522

(56) Entgegenhaltungen:
- WO-A-03/082290
- WO-A-2008/055854

## Beschreibung

Gegenstand der vorliegenden Erfindung sind Cyclohexyloxy-substituierte Heterocyclen der allgemeinen Formel 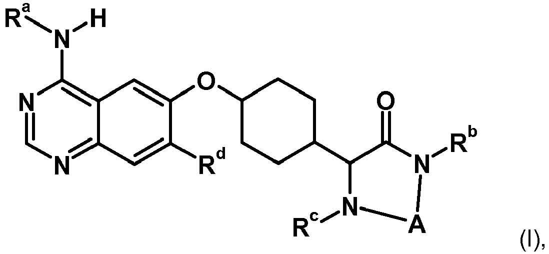 deren Tautomere, deren Stereoisomere, deren Gemische und deren Salze, insbesondere deren physiologisch verträgliche Salze mit anorganischen oder organischen Säuren, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege und deren Herstellung.

Aufgabe der vorliegenden Erfindung ist es, neue Verbindungen bereitzustellen, die aufgrund ihrer pharmazeutischen Wirksamkeit als Tyrosin-Kinase Hemmer zur Anwendung auf therapeutischem Gebiet, d.h. zur Behandlung von pathophysiologischen Prozessen, die durch Überfunktion von Tyrosinkinasen hervorgerufen werden, gelangen können.

### Detaillierte Beschreibung der Erfindung

Überraschenderweise wurde gefunden, dass die vorstehend genannte Aufgabe durch Verbindungen der Formel (I), worin die Reste **R**^{a} bis **R**^{d} und **A** die nachstehend genannten Bedeutungen haben, gelöst wird.

Die vorliegende Erfindung betrifft daher Verbindungen der allgemeinen Formel (I), 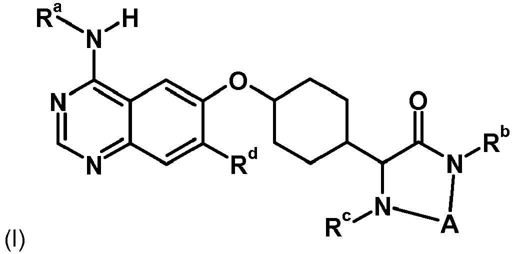 worin
- **R^{a}**: 3-Chlor-2-Fluor-phenyl-
- **R^{b}**: Wasserstoff, oder einen Rest, gegebenenfalls substituiert, ausgewählt aus der Gruppe bestehend aus C₁₋₆-Alkyl, C₃₋₆-Cycloalkyl- und C₃₋₆-Cycloalkyl-C₁₋₃-alkyl-,
- **R^{c}**: Wasserstoff, oder einen Rest, gegebenenfalls substituiert, ausgewählt aus der Gruppe bestehend aus C₁₋₆-Alkyl, C₃₋₆-Cycloalkyl-, C₃₋₆-Cycloalkyl-C₁₋₃-alkyl, C₁₋₆-Alkyl-CO-, C₃₋₆-Cycloalkyl-CO-, C₃₋₆-Cycloalkyl-C₁₋₃-alkyl-CO-, C₁₋₆-Alkyl-SO₂-, C₃₋₆-Cycloalkyl-SO₂-, C₃₋₆-Cycloalkyl-C₁₋₃-alkyl-SO₂-, Phenyl-CO- und Phenyl-SO₂-,
- **R^{d}**: , -O-Me
- **A**: , -CH₂-CH₂-
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate und Hydrate,
bedeuten.

Bevorzugt sind Verbindungen der Formel (I), worin
- **R^{b}** und **R^{c}**: gleich oder verschieden, Wasserstoff oder C₁₋₃-Alkyl,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, Solvate und Hydrate,
bedeuten.

Ein weiterer Gegenstand der Erfindung sind Verbindungen der Formel (I) zur Verwendung als Arzneimittel.

Bevorzugt ist die Verwendung der Verbindungen der Formel (I), wobei es sich um entzündliche oder allergische Erkrankungen der Atemwege handelt.

Besonders bevorzugt ist die Verwendung der Verbindungen der Formel (I), wobei es sich um eine Erkrankung handelt, die ausgewählt ist aus der Gruppe bestehend aus Chronischer Bronchitis, akuter Bronchitis, Bronchitis aufgrund bakterieller oder viraler Infektion oder Pilzen oder Helminthen, allergischer Bronchitis, toxischer Bronchitis, chronisch obstruktiver Bronchitis (COPD), Asthma (intrinsisch oder allergisch), pediatrischem Asthma, Bronchiektasien, allergischer Alveolitis, allergischer oder nicht-allergischer Rhinitis, chronischer Sinusitis, zystischer Fibrose oder Mukoviszidose, alpha-1-Antitrypsin-Mangel, Husten, Lungenemphysem, interstitieller Lungenerkrankungen, Alveolitis, hyperreaktiver Atemwege, Nasenpolypen, Lungenödemen, Pneumonitis aufgrund unterschiedlicher Genese wie Strahlen-induziert oder durch Aspiration oder infektiöse, Kollagenosen wie Lupus eryth, systemische Sklerodermie, Sarkoidose und M. Boeck.

Weiterhin bevorzugt ist die Verwendung der Verbindungen der Formel (I), wobei es sich um entzündliche oder allergische Krankheitszustände handelt, bei denen Autoimmun-Reaktionen beteiligt sind.

Weiterhin bevorzugt ist die Verwendung der Verbindungen der Formel (I), wobei es sich um eine Erkrankung in Form von benignen oder malignen Tumoren handelt.

Ein weiterer Gegenstand der Erfindung ist eine pharmazeutische Formulierung enthaltend eine Verbindung der Formel (I).

Bevorzugt ist eine oral applizierbare pharmazeutische Formulierung enthaltend eine Verbindung der Formel (I).

Ein weiterer Gegenstand der Erfindung ist eine Arzneimittelkombinationen, die neben einer oder mehrerer Verbindungen der Formel (I) als weiteren Wirkstoff einen oder mehrere Verbindungen enthalten, die ausgewählt sind aus den Klassen der Betamimetika, Anticholinergika, Corticosteroiden, weitere PDE4-Inhibitoren, LTD4-Antagonisten, EGFR-Hemmern, Dopamin-Agonisten, H1-Antihistaminika, PAF-Antagonisten und PI3-Kinase Inhibitoren oder zwei- oder dreifach Kombinationen davon.

Als Betamimetika gelangen hierbei vorzugsweise Verbindungen zur Anwendung, die ausgewählt sind aus der Gruppe bestehend aus Arformoterol, Carmoterol, Formoterol, Indacaterol, Salmeterol, Albuterole, Bambuterol, Bitolterol, Broxaterol, Carbuterol, Clenbuterol, Fenoterol, Hexoprenalin, Ibuterol, Isoetharin, Isoprenalin, Levosalbutamol, Mabuterol, Meluadrin, Metaproterenol, Milveterol, Orciprenalin, Pirbuterol, Procaterol, Reproterol, Rimiterol, Ritodrin, Salmefamol, Soterenol, Sulphonterol, Terbutalin, Tiaramid, Tolubuterol, Zinterol und
- 6-Hydroxy-8-{1-hydroxy-2-[2-(4-methoxy-phenyl)-1,1-dimethyl-ethylamino]-ethyl}-4H-benzo[1,4]oxazin-3-on
- 8-{2-[2-(2,4-Difluor-phenyl)-1,1-dimethyl-ethylamino]-1-hydroxy-ethyl}-6-hydroxy-4H-benzo[1,4]oxazin-3-on
- 8-{2-[2-(3,5-Difluor-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 8-{2-[2-(4-Fluor-phenyl)-1,1-dimethyl-ethylamino]-1-hydroxy-ethyl}-6-hydroxy-4H-benzo[1,4]oxazin-3-on
- N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[3-(4,4-Diethyl-6-fluoro-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid
- 8-{2-[1,1-Dimethyl-3-(2-oxo-2,3-dihydro-benzoimidazol-1-yl)-propylamino]-1-hydroxy-ethyl}-6-hydroxy-4H-benzo[1,4]oxazin-3-on
- 8-{2-[1,1-Dimethyl-3-(6-methyl-2-oxo-2,3-dihydro-benzoimidazol-1-yl)-propylamino]-1-hydroxy-ethyl}-6-hydroxy-4H-benzo[1,4]oxazin-3-on
- 8-{2-[1,1-Dimethyl-3-(2-oxo-5-trifluormethyl-2,3-dihydro-benzoimidazol-1-yl)-propylamino]-1-hydroxy-ethyl}-6-hydroxy-4H-benzo[1,4]oxazin-3-on
- 8-{2-[1,1-Dimethyl-3-(3-methyl-2-oxo-2,3-dihydro-benzoimidazol-1-yl)-propylamino]-1-hydroxy-ethyl}-6-hydroxy-4H-benzo[1,4]oxazin-3-on
- N-[2-Hydroxy-5-((1R)-1-hydroxy-2-{2-[4-(2-hydroxy-2-phenyl-ethylamino)-phenyl]-ethylamino}-ethyl)-phenyl]-formamid
- 8-Hydroxy-5-((1R)-1-hydroxy-2-{2-[4-(6-methoxy-biphenyl-3-ylamino)-phenyl]-ethylamino}-ethyl)-1 H-chinolin-2-on
- 8-Hydroxy-5-[(1R)-1-hydroxy-2-(6-phenethylamino-hexylamino)-ethyl]-1H-chinolin-2-on
- 5-[(1R)-2-(2-{4-[4-(2-Amino-2-methyl-propoxy)-phenylamino]-phenyl}-ethylamino)-1-hydroxy-ethyl]-8-hydroxy-1 H-chinolin-2-on
- [3-(4-{6-[(2R)-2-Hydroxy-2-(4-hydroxy-3-hydroxymethyl-phenyl)-ethylamino]-hexyloxy}-butyl)-5-methyl-phenyl]-harnstoff
- 4-((1R)-2-{6-[2-(2,6-Dichlor-benzyloxy)-ethoxy]-hexylamino}-1-hydroxy-ethyl)-2-hydroxymethyl-phenol
- 3-(4-{6-[(2R)-2-Hydroxy-2-(4-hydroxy-3-hydroxymethyl-phenyl)-ethylamino]-hexyloxy}-butyl)-benzenesulfonamid
- 3-(3-{7-[(2R)-2-Hydroxy-2-(4-hydroxy-3-hydroxymethyl-phenyl)-ethylamino]-heptyloxy}-propyl)-benzenesulfonamid
- 4-((1R)-2-{6-[4-(3-Cyclopentanesulfonyl-phenyl)-butoxy]-hexylamino}-1-hydroxy-ethyl)-2-hydroxymethyl-phenol
- *N*-1-Adamantanyl-2-{3-[(2R)-2-({(2R)-2-hydroxy-2-[4-hydroxy-3-(hydroxymethyl)phenyl]ethyl}amino)propyl]phenyl}acetamid
- (1R)-5-{2-[6-(2,2-Difluor-2-phenyl-ethoxy)-hexylamino]-1-hydroxy-ethyl}-8-hydroxy-1H-chinolin-2-on
- (R,S)-4-(2-{[6-(2,2-Difluor-4-phenylbutoxy)hexyl]amino}-1-hydroxy-ethyl)-2-(hydroxymethyl)phenol
- (R,S)-4-(2-{[6-(2,2-Difluor-2-phenylethoxy)hexyl]amino}-1-hydroxy-ethyl)-2-(hydroxymethyl)phenol
- (R,S)-4-(2-{[4,4-Difluor-6-(4-phenylbutoxy)hexyl]amino}-1-hydroxy-ethyl)-2-(hydroxymethyl)phenol
- (R,S)-4-(2-{[6-(4,4-Difluor-4-phenylbutoxy)hexyl]amino}-1-hydroxy-ethyl)-2-(hydroxymethyl)phenol
- (R,S)-5-(2-{[6-(2,2-Difluor-2-phenylethoxy)hexyl]amino}-1-hydroxy-ethyl)-8-hydroxychinolin-2(1 H)-on
- (R,S)-[2-({6-[2,2-Difluor-2-(3-methylphenyl)ethoxy]hexyl}amino)-1-hydroxyethyl]-2-(hydroxymethyl)phenol
- 4-(1R)-2-{[6-(2,2-Difluor-2-phenylethoxy)hexyl]amino}-1-hydroxyethyl)-2-(hydroxymethyl)phenol
- (R,S)-2-(Hydroxymethyl)-4-(1-hydroxy-2-{[4,4,5I5-tetrafluor-6-(3-phenylpropoxy)-hexyl]amino}ethyl)phenol
- (R,S)-[5-(2-{[6-(2,2-Difluor-2-phenylethoxy)hexyl]amino}-1-hydroxy-ethyl)-2-hydroxyphenyl]formamid
- (R,S)-4-[2-({6-[2-(3-Bromophenyl)-2,2-difluoroethoxy]hexyl}amino)-1-hydroxyethyl]-2-(hydroxymethyl)phenol
- (R, S)-N-[3-(1,1-Difluor-2-{[6-({2-hydroxy-2-[4-hydroxy-3-(hydroxymethyl)phenyl]-ethyl}amino)hexyl]oxy}ethyl)phenyl]-harnstoff
- 3-[3-(1,1-Difluor-2-{[6-({2-hydroxy-2-[4-hydroxy-3-(hydroxymethyl)phenyl]ethyl}-amino)hexyl]oxy}ethyl)phenyl]imidazolidin-2,4-dion
- (R,S)-4-[2-({6-[2,2-Difluor-2-(3-methoxyphenyl)ethoxy]hexyl}amino)-1-hydroxyethyl]-2-(hydroxymethyl)phenol
- 5-((1R)-2-{[6-(2,2-Difluor-2-phenylethoxy)hexyl]amino}-1-hydroxyethyl)-8-hydroxychinolin-2(1 H)-on
- 4-((1R)-2-{[4,4-Difluor-6-(4-phenylbutoxy)hexyl]amino}-1-hydroxy-ethyl)-2-(hydroxymethyl)phenol
- (R,S)-4-(2-{[6-(3,3-Difluor-3-phenylpropoxy)hexyl]amino}-1-hydroxy-ethyl)-2-(hydroxymethyl)phenol
- (R,S)-(2-{[6-(2,2-Difluor-2-phenylethoxy)-4,4-difluorohexyl]amino}-1-hydroxyethyl)-2-(hydroxymethyl)phenol
- (R,S)-4-(2-{[6-(2,2-Difluor-3-phenylpropoxy)hexyl]amino}-1-hydroxy ethyl)-2-(hydroxymethyl)phenol
- 3-[2-(3-Chlor-phenyl)-ethoxy]-N-(2-diethylamino-ethyl)-N-{2-[2-(4-hydroxy-2-oxo-2,3-dihydro-benzothiazol-7-yl)-ethylamino]-ethyl}-propionamid
- N-(2-Diethylamino-ethyl)-N-{2-[2-(4-hydroxy-2-oxo-2,3-dihydro-benzothiazol-7-yl)-ethylamino]-ethyl}-3-(2-naphthalen-1-yl-ethoxy)-propionamid
- 7-[2-(2-{3-[2-(2-Chlor-phenyl)-ethylamino]-propylsulfanyl}-ethylamino)-1-hydroxy-ethyl]-4-hydroxy-3H-benzothiazol-2-on
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus: Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Aclidiniumsalze, bevorzugt das Bromidsalz, Glycopyrroniumsalzen, bevorzugt das Bromidsalz, Trospiumsalzen, bevorzugt das Chloridsalz, Tolterodin, (3R)-1-Phenethyl-3-(9H-xanthen-9-carbonyloxy)-1-azoniabicyclo[2.2.2]octan-Salze . In den vorstehend genannten Salzen stellen die Kationen die pharmakologisch aktiven Bestandteile dar. Als Anionen X- können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt. Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.
- Als Corticosteroide gelangen hierbei vorzugsweise Verbindungen zur Anwendung, die ausgewählt sind aus der Gruppe bestehend aus: Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, Tipredane und Pregna-1,4-diene-3,20-dione, 6-fluoro-11-hydroxy-16,17-[(1-methylethylidene) bis(oxy)]-21-[[4-[(nitrooxy)methyl]benzoyl]oxy]-, (6-alpha,11-beta,16-alpha)- (9CI) (NCX-1024)
- 16,17-butylidenedioxy-6,9-difluoro-11-hydroxy-17-(methylthio)androst-4-en-3-one (RPR-106541),
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6-alpha,9-alpha-difluoro-11-beta-hydroxy-16alpha-methyl-3-oxo-17alpha-(2,2,3,3-tetramethylcyclopropylcarbonyl)oxy-androsta-1,4-diene-17beta-carbonsäure cyanomethyl ester,
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast Pumafentrin , Lirimilast , Apremilast, Arofyllin, Atizoram, Oglemilast, Tetomilast, und
- 5-[(N-(2,5-dichloro-3-pyridinyl)-carboxamid]-8-methoxy-Chinolin (D-4418),
- N-(3,5-dichloro-1-oxido-4-pyridinyl)-carboxamid]-8-methoxy-2-(trifluoromethyl)-Chinolin (D-4396 (Sch-351591)),N-(3,5-dichloropyrid-4-yl)-[1-(4-fluorobenzyl)-5-hydroxy-indol-3-yl]glyoxylsäureamid (AWD-12-281 (GW-842470)), 9-[(2-fluorophenyl)methyl]-N-methyl-2-(trifluoromethyl)-9H-Purin-6-amine (NCS-613),
- 4-[(2R)-2-[3-(cyclopentyloxy)-4-methoxyphenyl]-2-phenylethyl]-Pyridine (CDP-840),
- N-[(3R)-3,4,6,7-tetrahydro-9-methyl-4-oxo-1-phenylpyrrolo[3,2,1-jk][1,4]benzodiazepin-3-yl]-4-Pyridinecarboxamide (PD-168787),
- 4-[6,7-diethoxy-2,3-bis(hydroxymethyl)-1-naphthalenyl]-1-(2-methoxyethyl)-2(1H)-Pyridinone (T-440),
- 2-[4-[6,7-diethoxy-2,3-bis(hydroxymethyl)-1-naphthalenyl]-2-pyridinyl]-4-(3-pyridinyl)-1(2H)-Phthalazinone (T-2585),
- (3-(3-cyclopenyloxy-4-methoxybenzyl)-6-ethylamino-8-isopropyl-3H-purine (V-11294A),
- beta-[3-(cyclopentyloxy)-4-methoxyphenyl]-1,3-dihydro-1,3-dioxo-2H-Isoindole-2-propanamid (CDC-801),
- Imidazo[1,5-a]pyrido[3,2-e]pyrazin-6(5H)-one, 9-ethyl-2-methoxy-7-methyl-5-propyl- (D-22888)
- 5-[3-(cyclopentyloxy)-4-methoxyphenyl]-3-[(3-methylphenyl)methyl]-, (3S,5S)-2-Piperidinon (HT-0712),
- 4-[1-[3,4-bis(difluoromethoxy)phenyl]-2-(3-methyl-1-oxido-4-pyridinyl)ethyl]-alpha,alpha-bis(trifluoromethyl)-Benzenemethanol (L-826141)
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxyphenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als EGFR-Hemmer gelangen hierbei vorzugsweise Verbindungen zur Anwendung, die ausgewählt sind aus der Gruppe bestehend aus Cetuximab, Trastuzumab, Panitumumab ( = ABX-EGF), Mab ICR-62, Gefitinib, Canertinib, Erlotinib, und
- 4-[(3-Chlor-4-fluorphenyl)amino]-6-{[4-(morpholin-4-yl)-1-oxo-2-buten-1-yl]amino}-7-cyclopropylmethoxy-chinazolin,
- 4-[(3-Chlor-4-fluorphenyl)amino]-6-{[4-(N,N-diethylamino)-1-oxo-2-buten-1-yl]amino}-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopropylmethoxy-chinazolin,
- 4-[(R)-(1-Phenyl-ethyl)amino]-6-{[4-(morpholin-4-yl)-1-oxo-2-buten-1-yl]amino}-7-cyclopentyloxy-chinazolin,
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopentyloxy-chinazolin,
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin,
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin,
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin,
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin,
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin,
- 4-[(3-Ethinyl-phenyl)amino]-6,7-bis-(2-methoxy-ethoxy)-chinazolin,
- 4-[(3-Chlor-4-fluorphenyl)amino]-7-[3-(morpholin-4-yl)-propyloxy]-6-[(vinylcarbonyl)amino]-chinazolin,
- 4-[(R)-(1-Phenyl-ethyl)amino]-6-(4-hydroxy-phenyl)-7H-pyrrolo[2,3-d]pyrimidin,
- 3-Cyano-4-[(3-chlor-4-fluorphenyl)amino]-6-{[4-(N,N-dimethylamino)-1-oxo-2-buten-1-yl]amino}-7-ethoxy-chinolin,
- 4-{[3-Chlor-4-(3-fluor-benzyloxy)-phenyl]amino}-6-(5-{[(2-methansulfonyl-ethyl)amino]methyl}-furan-2-yl)chinazolin,
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
- 4-[(3-Ethinyl-phenyl)amino]-6-{[4-(5,5-dimethyl-2-oxo-morpholin-4-yl)-1-oxo-2-buten-1-yl]amino}-chinazolin,
- 4-[(3-Chlor-4-fluor-phenyl)amino]-6-[2-(2,2-dimethyl-6-oxo-morpholin-4-yl)-ethoxy]-7-methoxy-chinazolin,
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin,
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin,
- 4-[(3-Chlor-4-fluor-phenyl)amino]-6-{2-[4-(2-oxo-morpholin-4-yl)-piperidin-1-yl]-ethoxy}-7-methoxy-chinazolin,
- 4-[(3-Chlor-4-fluor-phenyl)amino]-6-[1-(tert.-butyloxycarbonyl)-piperidin-4-yloxy]-7-methoxy-chinazolin,
- 4-[(3-Chlor-4-fluor-phenyl)amino]-6-(trans-4-amino-cyclohexan-1-yloxy)-7-methoxy-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-(1-methyl-piperidin-4-yloxy)-7-methoxy-chinazolin,
- 4-[(3-Chlor-4-fluor-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(methoxymethyl)carbonyl]-piperidin-4-yloxy}-7-methoxy-chinazolin,
- 4-[(3-Chlor-4-fluor-phenyl)amino]-6-(piperidin-3-yloxy)-7-methoxy-chinazolin,
- 4-[(3-Chlor-4-fluor-phenyl)amino]-6-[1-(2-acetylamino-ethyl)-piperidin-4-yloxy]-7-methoxy-chinazolin,
- 4-[(3-Chlor-4-fluor-phenyl)amino]-6-(tetrahydropyran-4-yloxy)-7-ethoxy-chinazolin,
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin,
- 4-[(3-Chlor-4-fluor-phenyl)amino]-6-{trans-4-[(dimethylamino)sulfonylamino]-cyclohexan-1-yloxy}-7-methoxy-chinazolin,
- 4-[(3-Chlor-4-fluor-phenyl)amino]-6-{trans-4-[(morpholin-4-yl)carbonylamino]-cyclohexan-1-yloxy}-7-methoxy-chinazolin,
- 4-[(3-Chlor-4-fluor-phenyl)amino]-6-{trans-4-[(morpholin-4-yl)sulfonylamino]-cyclohexan-1-yloxy}-7-methoxy-chinazolin,
- 4-[(3-Chlor-4-fluor-phenyl)amino]-6-(tetrahydropyran-4-yloxy)-7-(2-acetylamino-ethoxy)-chinazolin,
- 4-[(3-Chlor-4-fluor-phenyl)amino]-6-(tetrahydropyran-4-yloxy)-7-(2-methansulfonylamino-ethoxy)-chinazolin,
- 4-[(3-Chlor-4-fluor-phenyl)amino]-6-{1-[(piperidin-1-yl)carbonyl]-piperidin-4-yloxy}-7-methoxy-chinazolin,
- 4-[(3-Chlor-4-fluor-phenyl)amino]-6-(1-aminocarbonylmethyl-piperidin-4-yloxy)-7-methoxy-chinazolin,
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin ,
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin,
- 4-[(3-Chlor-4-fluor-phenyl)amino]-6-(trans-4-ethansulfonylamino-cyclohexan-1-yloxy)-7-methoxy-chinazolin,
- 4-[(3-Chlor-4-fluor-phenyl)amino]-6-(1-methansulfonyl-piperidin-4-yloxy)-7-ethoxy-chinazolin,
- 4-[(3-Chlor-4-fluor-phenyl)amino]-6-(1-methansulfonyl-piperidin-4-yloxy)-7-(2-methoxy-ethoxy)-chinazolin,
- 4-[(3-Chlor-4-fluor-phenyl)amino]-6-[1-(2-methoxy-acetyl)-piperidin-4-yloxy]-7-(2-methoxy-ethoxy)-chinazolin,
- 4-[(3-Chlor-4-fluor-phenyl)amino]-6-(cis-4-acetylamino-cyclohexan-1-yloxy)-7-methoxy-chinazolin,
- 4-[(3-Ethinyl-phenyl)amino]-6-[1-(tert.-butyloxycarbonyl)-piperidin-4-yloxy]-7-methoxy-chinazolin,
- 4-[(3-Ethinyl-phenyl)amino]-6-(tetrahydropyran-4-yloxy]-7-methoxy-chinazolin,
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
- 4-[(3-Chlor-4-fluor-phenyl)amino]-6-{cis-4-[(morpholin-4-yl)carbonylamino]-cyclohexan-1-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Chlor-4-fluor-phenyl)amino]-6-{1-[(morpholin-4-yl)carbonyl]-piperidin-4-yloxy}-7-(2-methoxy-ethoxy)-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxy-chinazolin,
- 4-[(3-Chlor-4-fluor-phenyl)amino]-6-(1-methyl-piperidin-4-yloxy)-7(2-methoxy-ethoxy)-chinazolin,
- 4-[(3-Chlor-4-fluor-phenyl)amino]-6-(1-isopropyloxycarbonyl-piperidin-4-yloxy)-7-methoxy-chinazolin,
- 4-[(3-Chlor-4-fluor-phenyl)amino]-6-(cis-4-methylamino-cyclohexan-1-yloxy)-7-methoxy-chinazolin,
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin,
- 4-[(3-Ethinyl-phenyl)amino]-6-(piperidin-4-yloxy)-7-methoxy-chinazolin,
- 4-[(3-Ethinyl-phenyl)amino]-6-[1-(2-methoxy-acetyl)-piperidin-4-yloxy]-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin,
- 4-[(3-Chlor-4-fluor-phenyl)amino]-6-{1-[(2-methyl-morpholin-4-yl)carbonyl]-piperidin-4-yloxy}-7-methoxy-chinazolin,
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin,
- 4-[(3-Chlor-4-fluor-phenyl)amino]-6-{1-[(N-methyl-N-2-methoxyethyl-amino)carbonyl]-piperidin-4-yloxy}-7-methoxy-chinazolin,
- 4-[(3-Chlor-4-fluor-phenyl)amino]-6-(1-ethyl-piperidin-4-yloxy)-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-{1-[(3-methoxypropyl-amino)-carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxy-chinazolin,
- 4-[(3-Chlor-4-fluor-phenyl)amino]-6-(1-cyano-piperidin-4-yloxy)-7-methoxy-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin ;
- [4-[(3-chloro-4-fluoro-phenyl)amino]-6-{[4-(homomorpholin-4-yl)-1-oxo-2-buten-1-yl]amino}-7-[(S)-(tetrahydrofuran-3-yl)oxy]-quinazoline ,
- 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin,
- 4-[(3-Chlor-4-fluor-phenyl)amino]-7-[2-((S)-6-methyl-2-oxo-morpholin-4-yl)-ethoxy]-6-[(vinylcarbonyl)amino]-chinazolin,
- 4-[(3-Chlor-4-fluor-phenyl)amino]-7-[4-((R)-6-methyl-2-oxo-morpholin-4-yl)-butyloxy]-6-[(vinylcarbonyl)amino]-chinazolin,
- 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, und
- 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin,
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.
- Als LTD4-Rezeptor Antagonisten gelangen hierbei vorzugsweise Verbindungen zur Anwendung, die ausgewählt sind aus der Gruppe bestehend aus Montelukast, Pranlukast, Zafirlukast, und (E)-8-[2-[4-[4-(4-Fluorophenyl)butoxy]phenyl]ethenyl]-2-(1H-tetrazol-5-yl)-4H-1-benzopyran-4-one (MEN-91507)
- 4-[6-Acetyl-3-[3-(4-acetyl-3-hydroxy-2-propylphenylthio)propoxy]-2-propylphenoxy]-buttersäure (MN-001)
- 1-(((R)-(3-(2-(6,7-Difluor-2-chinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als Histamin H1 Rezeptor Antagonisten gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Olopatadine, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.
- Als PAF-Antagonisten gelangen hierbei vorzugsweise Verbindungen zur Anwendung, die ausgewählt sind aus der Gruppe bestehend aus Lexipafant und 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin
- 6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als Dopamin-Rezeptor Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als Substanzen bevorzugter PI3 Kinase Antagonisten gelangen hierbei vorzugsweise Verbindungen zur Anwendung, die ausgewählt sind aus der Gruppe bestehend aus
- 5-(Quinoxalin-6-ylmethylene)thiazolidine-2,4-dione (AS-605240),
- 2-[(6-amino-9H-purin-9-yl)methyl]-5-methyl-3-(2-methylphenyl)-4(3H)-Quinazolinone (C-87114),
- 2-Methyl-2-[4-[3-methyl-2-oxo-8-(chinolin-3-yl)-2,3-dihydroimidazo[4,5-c]chinolin-1-yl]phenyl]propionitrile (BEZ-235),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

### Verwendete Begriffe und Definitionen

Unter dem Begriff "gegebenenfalls substituiert" wird im Rahmen der Erfindung die genannte Gruppe verstanden, die gegebenenfalls mit einem niedermolekularen Rest substituiert ist. Als niedermolekulare Reste werden als chemisch sinnvoll anzusehende Gruppen verstanden, bestehend aus 1-25 Atomen. Bevorzugt haben solche Gruppen keinen negativen Effekt auf die pharmakologische Wirksamkeit der Verbindungen.

Beispielsweise können die Gruppen umfassen:
- Gerade oder verzweigte Kohlenstoffketten, gegebenenfalls unterbrochen durch Heteroatome, gegebenenfalls substituiert mit Ringen, Heteroatomen oder anderen gängigen funktionellen Gruppen.
- Aromatische oder nicht-aromatische Ringsysteme bestehend aus Kohlenstoffatomen und gegebenenfalls Heteroatomen, die wiederum substituiert sein können mit funktionellen Gruppen.
- Mehrere aromatische oder nicht-aromatische Ringsysteme bestehend aus Kohlenstoffatomen und gegebenenfalls Heteroatomen, die durch eine oder mehrere Kohlenstoffketten, gegebenenfalls unterbrochen durch Heteroatome, gegebenenfalls substituiert mit Heteroatomen oder anderen gängigen funktionellen Gruppen verknüpft sein können.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Sofern in der Strukturformel eines Substituenten ein einseitig offener Bindestrich "-" verwendet wird, ist dieser Bindestrich als Verknüpfungspunkt zum Rest des Moleküls zu verstehen. Der Substituent tritt an die Stelle der entsprechenden Reste **R^{a}, R^{b},** etc.. Sofern in der Bezeichnung oder Strukturformel eines Substituenten kein einseitig offener Bindestrich verwendet wird, ergibt sich der Verknüpfungspunkt zum Rest des Moleküls eindeutig aus der Bezeichnung oder Strukturformel selbst.

Verbindungen der allgemeinen Formel **(I)** können Säuregruppen besitzen, hauptsächlich Carboxylgruppen, und/oder basische Gruppen wie z.B. Aminofunktionen. Verbindungen der allgemeinen Formel **(I)** können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen. Zur Darstellung der Alkali- und Erdalkalimetallsalze der Verbindung der Formel **(I),** kommen vorzugsweise die Alkali- und Erdalkalihydroxide und - hydride in Betracht, wobei die Hydroxide und Hydride der Alkalimetalle, besonders des Natriums und Kaliums bevorzugt, Natrium- und Kaliumhydroxid besonders bevorzugt sind. (siehe auch Pharmaceutical salts, Birge, S.M. et al., J. Pharm. Sci., (1977), 66, 1-19) Wie vorstehend genannt, können die Verbindungen der allgemeinen Formel (I) in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen Diastereomeren, Mischungen der einzelnen Diastereomeren und/oder der einzelnen Enantiomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organischen Säuren - wie beispielsweise Weinsäure, Fumarsäure, Zitronensäure oder Methansulfonsäure.

"Schutzgruppen" in Sinne der vorliegenden Erfindung sind als Sammelbezeichnung für solche organische Reste zu verstehen, mit denen bestimmte funktionelle Gruppen eines mehrere aktive Zentren enthaltenden Moleküls vorübergehend gegen den Angriff von Reagenzien geschützt werden können, so dass Reaktionen nur an den gewünschten (ungeschützten) Stellen stattfinden. Die Schutzgruppen sollen unter milden Bedingungen selektiv einzuführen sein. Sie müssen für die Dauer des Schutzes unter allen Bedingungen der durchzuführenden Reaktionen und Reinigungsoperationen stabil sein; Racemisierungen und Epimerisierungen müssen unterdrückt werden. Schutzgruppen sollen wieder unter milden Bedingungen selektiv und idealerweise mit hoher Ausbeute abspaltbar sein. Die Wahl einer geeigneten Schutzgruppe, die Bedingungen zur Umsetzung (Lösungsmittel, Temperatur, Dauer, etc.) aber auch die Möglichkeiten eine Schutzgruppe wieder zu entfernen sind im Stand der Technik bekannt (z.B. Philip Kocienski, Protecting Groups, 3rd ed. 2004, THIEME, Stuttgart, ISBN: 3131370033).

Unter einem "organischen Lösungsmittel" wird im Rahmen der Erfindung ein organsicher, niedermolekularer Stoff verstanden, der andere organische Stoffe auf physikalischem Wege zur Lösung bringen kann. Voraussetzung für die Eignung als Lösungsmittel ist, dass sich beim Lösungsvorgang weder der lösende noch der gelöste Stoff chemisch verändern, dass also die Komponenten der Lösung durch physikalische Trennverfahren wie Destillation, Kristallisation, Sublimation, Verdunstung, Adsorption in der Originalgestalt wieder gewonnen werden können. Aus verschiedenen Gründen können nicht nur die reinen Lösungsmittel, sondern Gemische, die die Lösungseigenschaften vereinigen verwendet werden. Beispielsweise seinen genannt:
- Alkohole, bevorzugt Methanol, Ethanol, Propanol, Butanol, Octanol, Cyclohexanol;
- Glykole, bevorzugt Ethylenglykol, Diethylenglykol;
- Ether / Glykolether, bevorzugt Diethylether, tert-Butylmethylether, Dibutylether, Anisol, Dioxan, Tetrahydrofuran, Mono-, Di-, Tri-, Polyethylenglykolether;
- Ketone, bevorzugt Aceton, Butanon, Cyclohexanon;
- Ester, bevorzugt Essigsäureester, Glykolester;
- Amide u.a. Stickstoff-Verbindungen, bevorzugt Dimethylformamid, Pyridin, N-Methylpyrrolidon, Acetonitril;
- Schwefel-Verbindungen, bevorzugt Schwefelkohlenstoff, Dimethylsulfoxid, Sulfolan;
- Nitro-Verbindungen bevorzugt Nitrobenzol;
- Halogenkohlenwasserstoffe, bevorzugt Dichlormethan, Chloroform, Tetrachlormethan, Tri-, Tetrachlorethen, 1,2-Dichlorethan, Chlorfluorkohlenstoffe;
- aliphatische oder alicyclische Kohlenwasserstoffe, bevorzugt Benzine, Petrolether, Cyclohexan, Methylcyclohexan, Decalin, Terpen-L.; oder
- aromatische Kohlenwasserstoffe, bevorzugt Benzol, Toluol, *o*-Xylol, *m*-Xylol, *p*-Xylol;
oder entsprechende Gemische davon.

Die Bezeichnung diastereomerenrein beschreibt im Rahmen der vorliegenden Erfindung Verbindungen der Formel (I), die in einer Diastereomerenreinheit von wenigstens 85%de, bevorzugt von wenigstens 90%de, besonders bevorzugt von > 95%de vorliegen. Die Bezeichnung de (diastereomeric excess) ist im Stand der Technik bekannt und beschreibt den optischen Reinheitsgrad diastereomerer Verbindungen.

Die Bezeichnung enantiomerenrein beschreibt im Rahmen der vorliegenden Erfindung Verbindungen der Formel (I), die in einer Enantiomerenreinheit von wenigstens 85%ee, bevorzugt von wenigstens 90%ee, besonders bevorzugt von > 95%ee vorliegen. Die Bezeichnung ee (enantiomeric excess) ist im Stand der Technik bekannt und beschreibt den optischen Reinheitsgrad chiraler Verbindungen.

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatomen, besonders bevorzugt Alkylgruppen mit 1 bis 2 Kohlenstoffatomen. Beispielsweise werden hierfür genannt: Methyl, Ethyl, *n*-Propyl, *iso*-Propyl, *n*-Butyl, *iso*-Butyl, *sec*-Butyl, *tert*-Butyl, *n-*Pentyl, *iso*-Pentyl, *neo*-Pentyl oder Hexyl. Gegebenenfalls werden für die vorstehend genannten Gruppen auch die Abkürzungen Me, Et, n-Pr, *i*-Pr, n-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso-*Propyl, Butyl umfasst *iso*-Butyl, *sec*-Butyl und *tert*-Butyl etc.

Unter dem Begriff "C₁₋₃-Alkylen" (auch soweit sie Bestandteil anderer Reste sind) werden verzweigte und unverzweigte Alkylengruppen mit 1 bis 3 Kohlenstoffatomen verstanden. Bevorzugt sind Alkylengruppen mit 1 bis 2 Kohlenstoffatomen. Beispielsweise werden hierfür genannt: Methylen, Ethylen, Propylen und 1-Methylethylen. Sofern nicht anders beschrieben, umfasst die Definition "Propylen" alle denkbaren isomeren Formen der jeweiligen Reste gleicher Kohlenstoffanzahl. So umfasst beispielsweise Propylen auch 1-Methylethylen.

Unter dem Begriff "C₂₋₃-Alkenyl" (auch soweit sie Bestandteil anderer Reste sind) werden Alkenylgruppen mit 2 bis 3 Kohlenstoffatomen verstanden, soweit sie mindestens eine Doppelbindung aufweisen. Beispielsweise werden hierfür genannt: Ethenyl bzw. Vinyl. Sofern nicht anders beschrieben, umfasst die Definition "C₂₋₃-Alkenyl" alle denkbaren isomeren Formen der jeweiligen Reste gleicher Kohlenstoffanzahl. So umfasst beispielsweise Propenyl 1-Propen-1-yl, 1-Propen-2-yl und 2-Propen-1-yl.

Unter dem Begriff "C₂₋₃-Alkinyl" (auch soweit sie Bestandteil anderer Reste sind) werden Alkinylgruppen mit 2 bis 3 Kohlenstoffatomen verstanden, soweit sie mindestens eine Dreifachbindung aufweisen. Beispielsweise werden hierfür genannt: Ethinyl oder Propinyl. Sofern nicht anders beschrieben, umfasst die Definition "C₂₋₃-Alkinyl" alle denkbaren isomeren Formen der jeweiligen Reste gleicher Kohlenstoffanzahl. So umfasst beispielsweise Propinyl 1-Propin-1-yl und 2-Propin-1-yl.

Unter dem Begriff "C₃₋₇-Cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert-*Butyl, Hydroxy und Fluor.

Unter dem Begriff "Aryl" (auch soweit sie Bestandteil anderer Reste sind) werden aromatische Ringsysteme mit 6, 10 oder 14 Kohlenstoffatomen verstanden. Beispielsweise werden hierfür genannt: Phenyl, Naphthyl, Anthracenyl oder Phenanthrenyl, bevorzugter Arylrest ist Phenyl. Soweit nicht anders beschrieben, können die Aromaten substituiert sein mit einem oder mehreren Resten **R⁵.**

Insbesondere bevorzugt ist unter dem Begriff "Aryl" jeweils eine Phenylgruppe zu verstehen, die durch **R⁵** mono- oder disubstituiert ist, wobei die Substituenten **R⁵** gleich oder verschieden sein können und
- **R⁵**: Wasserstoff, oder einen Rest, gleich oder verschieden, ausgewählt aus der Gruppe bestehend aus F, Cl, Br, I, OH, CN, C₁₋₃-Alkyl-, C₁₋₃-Alkyl-O-, CHF₂, CF₃, -O-CHF₂ und -O-CF₃, bedeutet.

Unter dem Begriff "Heteroaryl" werden 5-10-gliedrige mono- oder bicyclische Heteroarylringe, in denen bis zu drei C-Atome durch ein oder mehrere Heteroatome ausgewählt aus der Gruppe Sauerstoff, Stickstoff oder Schwefel ersetzt sein können bezeichnet, wobei diese so viele konjugierte Doppelbindungen enthalten, dass ein aromatisches System gebildet wird. Jeder der vorstehend genannten Heterocyclen kann gegebenenfalls ferner an einen Benzolring anneliert sein. Die Heteroarylringe können, soweit nicht anders beschrieben, beispielsweise einen oder mehrere Substituenten tragen.

Der Ring kann über ein Kohlenstoffatom oder falls vorhanden über ein Stickstoffatom mit dem Molekül verknüpft sein. Als Beispiele für fünf- oder sechsgliedrige heterocyclische Aromaten, werden genannt:

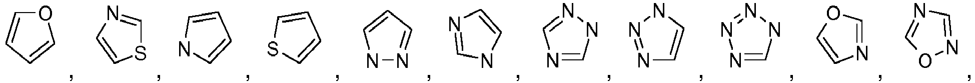

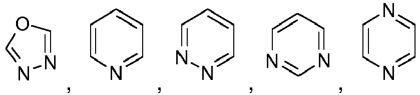

Als Beispiel für 5-10-gliedrige bicyclische Heteroarylringe werden genannt Pyrrolizin, Indol, Indolizin, Isoindol, Indazol, Purin, Chinolin, Isochinolin, Chinoxalin, Benzimidazol, Benzofuran, Benzothiophen, Benzothiazol, Benzoisothiazol, Pyridopyrimidin, Pteridin, Pyrimidopyrimidin. Insbesondere bevorzugt ist unter dem Begriff "Heteroaryl"
eine Pyridinyl-, Pyridazinyl-, Pyrimidinyl- oder Pyrazinyl-gruppe zu verstehen, wobei diese jeweils durch den Rest **R⁵** mono- oder disubstituiert sind, wobei die Substituenten **R⁵** gleich oder verschieden sein können und **R⁵** wie vorstehend erwähnt definiert ist.

"Halogen" steht im Rahmen der vorliegenden Erfindung für Fluor, Chlor, Brom oder Iod. Sofern nicht gegenteilig angegeben, gelten Fluor, Chlor und Brom als bevorzugte Halogene. . Ganz besonders bevorzugt bedeutet der Substituent **R^{a}** eine 3-Chlor-2-fluor-phenyl-Gruppe.

Der Substituent **R^{b}** kann Wasserstoff, oder einen Rest, gegebenenfalls substituiert, ausgewählt aus der Gruppe bestehend aus C₁₋₆-Alkyl, C₃₋₆-Cycloalkyl- und C₃₋₆-Cycloalkyl-C₁₋₃-alkyl, vorzugsweise Wasserstoff und C₁₋₃-Alkyl, insbesondere bevorzugt Wasserstoff und Methyl, bedeuten.

Der Substituent **R^{c}** kann Wasserstoff, oder einen Rest, gegebenenfalls substituiert, ausgewählt aus der Gruppe bestehend aus C₁₋₆-Alkyl, C₃₋₆-Cycloalkyl- und C₃₋₆-Cycloalkyl-C₁₋₃-alkyl, C₁₋₆-Alkyl-CO-, C₃₋₆-Cycloalkyl-CO- und C₃₋₆-Cycloalkyl-C₁₋₃-alkyl-CO-, C₁₋₆-Alkyl-SO₂-, C₃₋₆-Cycloalkyl-SO₂- und C₃₋₆-Cycloalkyl-C₁₋₃-alkyl-SO₂-, Phenyl-CO- und Phenyl-SO₂-, vorzugsweise Wasserstoff und C₁₋₃-Alkyl, insbesondere bevorzugt Wasserstoff und Methyl, bedeuten.

Der Substituent **R^{d}** kann insbesondere bevorzugt CH₃-O- bedeuten.

Eine besonders bevorzugte Bedeutung für **A** ist -CH₂CH₂-.

### Herstellungsverfahren

Zur Herstellung von Verbindungen der allgemeinen Formel (I) sind beispielsweise folgende Verfahren geeignet:
a) Umsetzung einer Verbindung der allgemeinen Formel 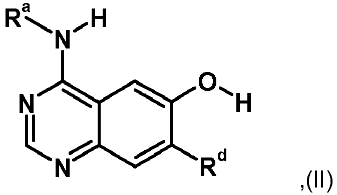 in der
   **R^{a}** und **R^{d}** wie eingangs erwähnt definiert sind, mit einer Verbindung der allgemeinen Formel 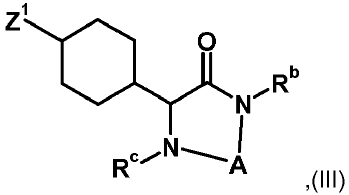 in der
   **R^{b}, R^{c}** und **A** wie eingangs erwähnt definiert ist und Z¹ eine Austrittsgruppe wie ein Halogenatom, z.B. ein Chlor- oder Bromatom, eine Sulfonyloxygruppe wie eine Methansulfonyloxy- oder p-Toluolsulfonyloxygruppe oder eine Hydroxygruppe darstellt.
   Mit einer Verbindung der allgemeinen Formel (III), in der Z¹ ein Halogenatom oder eine Sulfonyloxygruppe darstellt, erfolgt die Umsetzung zweckmäßigerweise in einem Lösungsmittel wie Ethanol, Isopropanol, Acetonitril, Toluol, Tetrahydrofuran, Dioxan, Dimethylformamid, Dimethylsulfoxid oder N-Methylpyrrolidinon, vorzugsweise in Gegenwart einer Base wie Kaliumcarbonat, Kalium-tert-butylat, Natriumhydrid oder N-Ethyl-diisopropylamin, bei Temperaturen im Bereich von 20°C bis 160°C, beispielsweise bei Temperaturen im Bereich von 60°C bis 140°C.
   Mit einer Verbindung der allgemeinen Formel III, in der Z¹ eine Hydroxygruppe darstellt, wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester, zweckmäßigerweise in einem Lösungsmittel wie Methylenchlorid, Acetonitril, Tetrahydrofuran, Dioxan, Toluol oder Ethylenglycoldiethylether bei Temperaturen zwischen -50 und 150°C, vorzugsweise jedoch bei Temperaturen zwischen -20 und 80°C, durchgeführt.
b) Zur Herstellung von Verbindungen der allgemeinen Formel I, in der **R^{b}** und **R^{c}** jeweils ein Wasserstoffatom und **A** eine -CO- Gruppe bedeuten, Umsetzung einer Verbindung der allgemeinen Formel 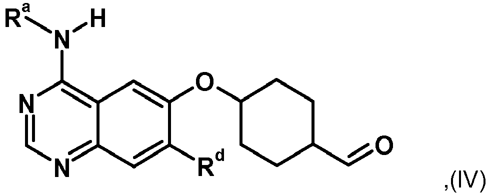 in der
   **R^{a}** und **R^{d}** wie eingangs erwähnt definiert sind, mit einem Alkalimetallcyanid und Ammoniumcarbonat.
   Die Reaktion wird beispielsweise in einem Lösemittel oder Lösemittelgemisch wie Methanol, Ethanol, Ethanol/Wasser oder Isopropanol bei Temperaturen zwischen Raumtemperatur und 120°C durchgeführt. Weitere Hinweise zur Synthese von Hydantoinen finden sich beispielsweise in der folgenden Publikation:
   Meusel, M.; Guetschow, M., Organic Preparations and Procedures International (2004), 36(5), 391-443.
c) Umsetzung einer Verbindung der allgemeinen Formel (V) 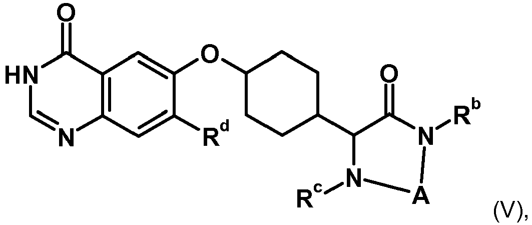 in der **R^{b}, R^{c}, R^{d}** und **A** wie eingangs erwähnt definiert sind, mit einem Halogenierungsmittel, beispielsweise einem Säurehalogenid wie Thionylchlorid, Thionylbromid, Phosphortrichlorid, Phosphorpentachlorid, Phosphoroxychlorid, oder Triphenylphosphin/Tetrachlorkohlenstoff oder Triphenylphosphin/N-Chlorsuccinimid zu einer Zwischenverbindung der allgemeinen Formel (VI), 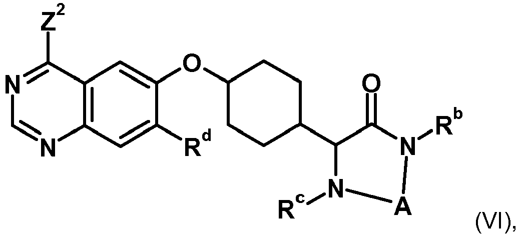 in der **R^{b}, R^{c}, R^{d}** und **A** wie eingangs erwähnt definiert sind und Z² ein Halogenatom wie ein Chlor- oder Bromatom darstellt,
   und anschließender Umsetzung mit einer Verbindung der allgemeinen Formel (VII),
   **R^{a}**-NH₂ (VII), in der **R^{a}** wie eingangs erwähnt definiert ist, oder dessen Salzen.
   Die Umsetzung mit dem Halogenierungsmittel wird gegebenenfalls in einem Lösungsmittel wie Methylenchlorid, Chloroform, Acetonitril oder Toluol und gegebenfalls in Gegenwart einer Base wie N,N-Diethylanilin, Triethylamin oder N-Ethyl-diisopropylamin bei Temperaturen im Bereich von 20°C bis 160°C, vorzugsweise von 40°C bis 120°C durchgeführt. Vorzugsweise wird die Reaktion jedoch mit Thionylchlorid und katalytischen Mengen an Dimethylformamid bei der Siedetemperatur des Reaktionsgemisches oder mit Phosphoroxychlorid in Acetonitril in Gegenwart von Triethylamin bei der Siedetemperatur des Reaktionsgemisches oder mit Triphenylphosphin/Tetrachlorkohlenstoff oder mit Triphenylphosphin/N-Chlorsuccinimid in Acetonitril durchgeführt.
   Die Umsetzung der Verbindung der allgemeinen Formel (VI) mit der Verbindung der allgemeinen Formel (VII) oder deren Salzen erfolgt zweckmäßigerweise in einem Lösungsmittel wie Ethanol, Isopropanol, Acetonitril, Dioxan oder Dimethylformamid, gegebenfalls in Gegenwart einer Base wie Kaliumcarbonat, Triethylamin oder N-Ethyl-diisopropylamin, bei Temperaturen im Bereich von 20°C und 160°C, vorzugsweise von 60°C bis 120°C. Vorzugsweise wird die Reaktion jedoch in Isopropanol bei der Siedetemperatur des Reaktionsgemisches durchgeführt.
   Die Umsetzung einer Verbindung der allgemeinen Formel (V) zu einer Verbindung der allgemeinen Formel (I) kann auch als Eintopfreaktion, beispielsweise in Acetonitril in Gegenwart von Triethylamin, durchgeführt werden.
d) Zur Herstellung von Verbindungen der allgemeinen Formel I, in der **R^{d}** eine der eingangs erwähnten, gegebenenfalls substituierten Alkyloxygruppen darstellt:
   Umsetzung einer Verbindung der allgemeinen Formel 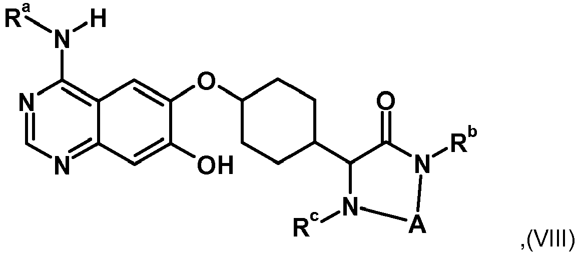 in der **R^{a}, R^{b}, R^{c}** und **A** wie eingangs erwähnt definiert sind, mit einer Verbindung der allgemeinen Formel

      Z³ - R^{d'}, (IX)

      in der **R^{d'}** einen Rest bedeutet ausgewählt aus der Gruppe bestehend aus C₁₋₄-Alkyl, mit 1 bis 3 Fluoratomen substituiertes C₁₋₂-Alkyl-, C₃₋₇₋Cycloalkyl-, C₃₋₇₋Cycloalkyl-C₁₋₄-alkyl-, Tetrahydrofuran-3-yl-, Tetrahydropyran-3-yl-, Tetrahydro-pyran-4-yl-, Tetrahydrofuranyl-C₁₋₄-alkyl- und Tetrahydropyranyl-C₁₋₄-alkyl-,
      oder
      R⁴-C₂₋₄-alkyl-, wobei der Rest **R⁴** durch mindestens 2 C-Atome von Z³ getrennt ist,
      oder
      einen Rest bedeutet ausgewählt aus der Gruppe bestehend aus Pyrrolidin-2-yl-C₁₋₄-alkyl-, Pyrrolidin-3-yl-C₁₋₄-alkyl-, Piperidin-2-yl-C₁₋₄-alkyl-, Piperidin-3-yl-C₁₋₄-alkyl-, Piperidin-4-yl-C₁₋₄-alkyl-, Azepan-2-yl-C₁₋₄-alkyl-, Azepan-3-yl-C₁₋₄-alkyl-, Azepan-4-yl-C₁₋₄-alkyl-, Morpholin-2-yl-C₁₋₄-alkyl-, Morpholin-3-yl-C₁₋₄-alkyl-, 1-(C₁₋₃-Alkyl)-pyrrolidin-2-yl-C₁₋₄-alkyl-, 1-(C₁₋₃-Alkyl)-pyrrolidin-3-yl-C₁₋₄-alkyl-, 1-(C₁₋₃-Alkyl)-piperidin-2-yl-C₁₋₄-alkyl-, 1-(C₁₋₃-Alkyl)-piperidin-3-yl-C₁₋₄-alkyl-, 1-(C₁₋₃-Alkyl)-piperidin-4-yl-C₁₋₄-alkyl-, 1-(C₁₋₃-Alkyl)-azepan-2-yl-C₁₋₄-alkyl-, 1-(C₁₋₃-Alkyl)-azepan-3-yl-C₁₋₄-alkyl-, 1-(C₁₋₃-Alkyl)-azepan-4-yl-C₁₋₄-alkyl-, 4-(C₁₋₃-Alkyl)-morpholin-2-yl-C₁₋₄-alkyl-, 4-(C₁₋₃-Alkyl)-morpholin-3-yl-C₁₋₄-alkyl-,
      und
      Z³ eine Austrittsgruppe wie ein Halogenatom, eine Alkylsulfonyloxy-, Arylsulfonyloxy- oder eine Hydroxygruppe darstellt.
      Handelt es sich bei der Austrittsgruppe um ein Halogenatom wie ein Chlor-, Brom- oder lodatom oder um eine Alkylsulfonyloxy- oder Arylsulfonyloxygruppe wie die die Methansulfonyloxy oder p-Toluolsulfonyloxygruppe, wird die Reaktion vorzugsweise in Gegenwart einer organischen oder anorganischen Base wie Kaliumcarbonat, Natriumhydrid oder N-Ethyl-diisopropylamin durchgeführt. Handelt es sich bei der Austrittsgruppe um eine Hydroxygruppe, so wird die Umsetzung in Gegenwart eines wasserentziehenden Mittels, vorzugsweise in Gegenwart eines Phosphins und eines Azodicarbonsäurederivates wie z.B. Triphenylphosphin/Azodicarbonsäurediethylester durchgeführt.
e) Zur Herstellung von Verbindungen der allgemeinen Formel I, in der **R^{d}** eine R^{4'}-C₂₋₄-alkyl-O- Gruppe darstellt, wobei der Rest R^{4'} durch mindestens 2 C-Atome von dem Sauerstoffatom getrennt ist, und R^{4'} einen Rest bedeutet ausgewählt aus der Gruppe bestehend aus NH₂, C₁₋₃-Alkyl-NH-, (C₁₋₃-Alkyl)₂N-, (2-Methoxyethyl)₂N-, Pyrrolidin-1-yl-, Piperidin-1-yl-, Azepan-1-yl-, Morpholin-4-yl-, 1,4-Oxazepan-4-yl-, 2-Oxa-5-aza-bicyclo[2.2.1]hept-5-yl-, 3-Oxa 8-aza-bicyclo[3.2.1]oct-8-yl-, 8-Oxa-3-aza-bicyclo[3.2.1]oct-3-yl-, Piperazin-1-yl-, 4-(C₁₋₃-Alkyl) piperazin-1-yl-, 1,4-Diazepan-1-yl-, 4-(C₁₋₃-Alkyl)-1,4-diazepan-1-yl:
   Umsetzung einer Verbindung der allgemeinen Formel 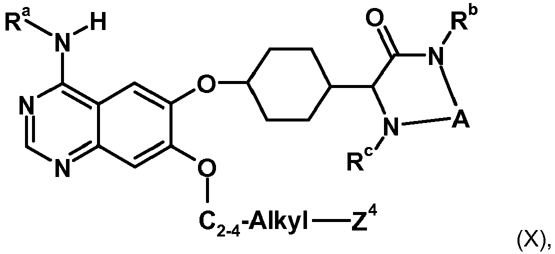 in der **R^{a}, R^{b}, R^{c}** und **A** wie eingangs erwähnt definiert sind und Z⁴ eine Austrittsgruppe wie ein Halogenatom, z.B. ein Chlor- oder Bromatom oder eine Sulfonyloxygruppe wie eine Methansulfonyloxy- oder p-Toluolsulfonyloxygruppe darstellt, mit

      H - R^{4'}, (XI)

      wobei R⁴ wie vorstehend erwähnt definiert ist.
f) Zur Herstellung von Verbindungen der allgemeinen Formel I, in der R^{b} ein Wasserstoffatom bedeutet:
   Abspaltung eines Schutzrestes von einer Verbindung der allgemeinen Formel 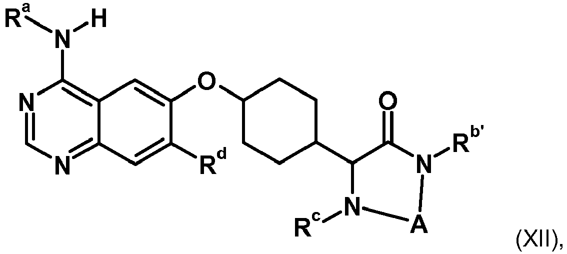
   in der **R^{a}, R^{c}, R^{d}** und A wie eingangs erwähnt definiert sind und R^{b'} eine Schutzgruppe bedeutet, beispielsweise eine gegebenenfalls substituierte Benzylgruppe, eine tert.-Butylgruppe oder eine 2-(Trimethylsilyl)ethyl-Gruppe.
   Die Abspaltung einer gegebenenfalls substituierten Benzyl-Gruppe erfolgt beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol, Thioanisol, Pentamethylbenzol oder Triethylsilan.
   Die Abspaltung eines gegebenenfalls substituierten Benzyl-Restes oder eines tert.-Butyl-Restes kann beispielsweise auch durch Behandlung mit einer Säure wie Trifluoressigsäure, Salzsäure oder Bromwasserstoffsäure, gegebenenfalls unter Verwendung eines Lösungsmittels wie Methylenchlorid oder Toluol, gegebenenfalls in Gegenwart von Anisol, Thioanisol, Pentamethylbenzol oder Triethylsilan erfolgen.
   Die Spaltung einer 2-(Trimethylsilyl)ethyl-Gruppe erfolgt beispielsweise durch Behandlung mit Fluoriden wie Tetrabutylammoniumfluorid gegebenenfalls unter Verwendung eines Lösungsmittels wie Tetrahydrofuran oder Dioxan.
   Weitere geeignete Schutzgruppen und Möglichkeiten zu ihrer Einführung und Abspaltung sind beispielsweise in "Protective Groups in Organic Synthesis" von Theodora W. Greene und Peter G. M. Wuts , Wiley-VCH, oder Philip Kocienski, Protecting Groups, 3rd ed. 2004, THIEME beschrieben.
g) Zur Herstellung von Verbindungen der allgemeinen Formel I, in der **R^{c}** ein Wasserstoffatom bedeutet:
   Abspaltung eines Schutzrestes von einer Verbindung der allgemeinen Formel 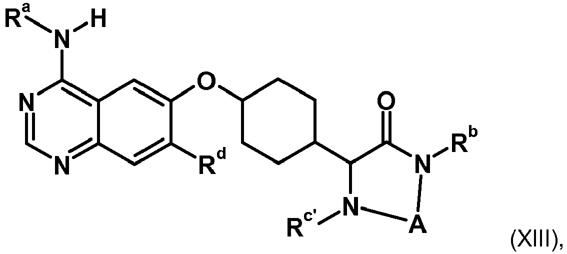
   in der **R^{a}, R^{b}, R^{d}** und **A** wie eingangs erwähnt definiert sind und R^{c'} eine Schutzgruppe bedeutet, beispielsweise eine gegebenenfalls substituierte Benzylgruppe oder eine Formyl-, Acetyl-, Trifluoracetyl-, Methoxycarbonyl-, Ethoxycarbonyl-, tert.-Butoxycarbonyl- oder Benzyloxycarbonyl-Gruppe.
   Die Abspaltung des Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von lodtrimethylsilan, bei Temperaturen zwischen 0 und 120°C, vorzugsweise bei Temperaturen zwischen 10 und 100°C.
   Die Abspaltung einer gegebenenfalls substituierten Benzyl-Gruppe, oder eines Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar.
   Die Abspaltung eines tert.-Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Methanol oder Diethylether.
   Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.
   Weitere geeignete Schutzgruppen und Möglichkeiten zu ihrer Einführung und Abspaltung sind beispielsweise in "Protective Groups in Organic Synthesis" von Theodora W. Greene und Peter G. M. Wuts , Wiley-VCH, oder Philip Kocienski, Protecting Groups, 3rd ed. 2004, THIEME beschrieben.
h) Zur Herstellung von Verbindungen der allgemeinen Formel I, in der **A** eine -C₂-C₃-Alkylen-Gruppe bedeutet:
   Cyclisierung einer Verbindung der allgemeinen Formel 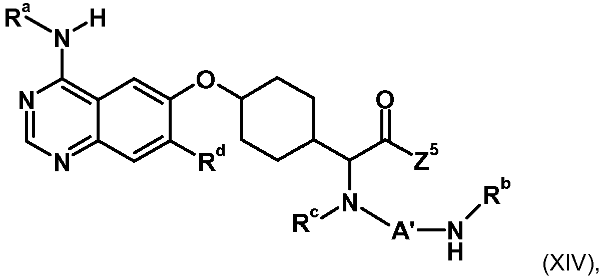
   in der **R^{a}, R^{b}, R^{c}** und **R^{d}** wie eingangs erwähnt definiert sind, A' eine -C₂-C₃-Alkylen-Gruppe und Z⁵ eine Austrittsgruppe wie ein Halogenatom, eine Hydroxy- oder Alkyloxygruppe bedeutet.

Handelt es sich bei der Austrittsgruppe um eine Hydroxygruppe, so wird die Umsetzung in Gegenwart eines wasserentziehenden Mittels wie N,N'-Carbonyldiimidazol, N,N'-Dicyclohexyl-carbodiimid, O-(Benzotriazol-1-yl)-N,N,N'N'-tetramethyluronium-tetrafluoroborat (TBTU) oder O-(7-Azabenzotriazol-1-yl)-N,N,N',N'-tetramethyluronium-hexafluorophosphat (HATU), zweckmäßigerweise in einem Lösungsmittel wie Methylenchlorid,N,N-Dimethylformamid, Acetonitril, Tetrahydrofuran, Dioxan oder Ethylenglycoldiethylether bei Temperaturen zwischen -50°C und 100°C, vorzugsweise jedoch bei Temperaturen zwischen -20°C und 60°C, durchgeführt.

Handelt es sich bei der Austrittsgruppe um ein Halogenatom, so wird die Umsetzung vorzugsweise in Gegenwart einer Base wie Triethylamin oder N-Ethyl-diisopropylamin zweckmäßigerweise in einem Lösungsmittel wie Methylenchlorid, N,N-Dimethylformamid, Acetonitril, Tetrahydrofuran, Dioxan oder Ethylenglycoldiethylether bei Temperaturen zwischen -50°C und 100°C, vorzugsweise jedoch bei Temperaturen zwischen -20°C und 60°C, durchgeführt.

Handelt es sich bei der Austrittsgruppe um ein Alkyloxygruppe, so wird die Umsetzung gegebenenfalls in Gegenwart einer Base wie Kaliumcarbonat, Natriumhydroxid, Triethylamin oder N-Ethyl-diisopropylamin zweckmäßigerweise in einem Lösungsmittel wie Methanol, Ethanol, Isopropanol, Methylenchlorid, N,N-Dimethylformamid, Acetonitril, Tetrahydrofuran, Dioxan oder Ethylenglycoldiethylether bei Temperaturen zwischen -50°C und 120°C, vorzugsweise jedoch bei Temperaturen zwischen 0°C und 80°C, durchgeführt.

Erhält man erfindungsgemäß eine Verbindung der allgemeinen Formel I, die eine Amino-, Alkylamino- oder Iminogruppe enthält, so kann diese mittels Acylierung oder Sulfonylierung in eine entsprechende Acyl- oder Sulfonylverbindung der allgemeinen Formel I übergeführt werden, wobei als Acylierungsmittel beispielsweise Carbonsäurehalogenide, Carbonsäureanhydride und Carbonsäuren mit Aktivierungsmitteln wie N,N'-Carbonyldiimidazol, N,N'-Dicyclohexylcarbodiimid oder O-(Benzotriazol-1-yl)-N,N,N'N'-tetramethyluroniumtetrafluoroborat und als Sulfonylierungsmittel Sulfonylhalogenide in Frage kommen, und/oder
eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden und/oder
eine Verbindung der allgemeinen Formel I, die eine Alkoxycarbonylgruppe enthält, so kann diese durch Esterspaltung in eine Carbonsäure übergeführt werden und/oder
eine Verbindung der allgemeinen Formel I, die eine Alkoxycarbonylgruppe enthält, so kann diese durch Umsetzung mit einem Amin in ein Carbonsäureamid-Derivat übergeführt werden und/oder
eine Verbindung der allgemeinen Formel I, die eine Carboxy-Gruppe enthält, so kann diese durch Umsetzung mit einem Amin in ein Carbonsäureamid-Derivat übergeführt werden.

Bei den vorstehend beschriebenen Umsetzungen können gegebenenfalls vorhandene reaktive Gruppen wie Hydroxy-, Amino-, Alkylamino- oder Iminogruppen während der Umsetzung durch übliche Schutzgruppen geschützt werden, welche nach der Umsetzung wieder abgespalten werden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von lodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol, Thioanisol, Pentamethylbenzol oder Triethylsilan.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Weitere geeignete Schutzgruppen und Möglichkeiten zu ihrer Einführung und Abspaltung sind beispielsweise in "Protective Groups in Organic Synthesis" von Theodora W. Greene und Peter G. M. Wuts , Wiley-VCH, oder Philip Kocienski, Protecting Groups, 3rd ed. 2004, THIEME beschrieben.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren oder Basen übergeführt werden. Als Säuren kommen hierfür beispielsweise Salzsäure, Bromwasserstoffsäure, Schwefelsäure, Methansulfonsäure, Ethansulfonsäure, Benzolsulfonsäure, p-Toluolsulfonsäure, Phosphorsäure, Fumarsäure, Bernsteinsäure, Benzoesäure, Salicylsäure, Mandelsäure, Milchsäure, Malonsäure, Zitronensäure, L-Äpfelsäure, L-Weinsäure oder Maleinsäure in Betracht. Als Basen kommen hierfür beispielsweise Natronlauge, Kalilauge, Calciumhydroxid, Diethanolamin oder N-Methyl-D-glucamine in Betracht.

Die als Ausgangsstoffe verwendeten Verbindungen der allgemeinen Formeln II bis XXIII sind teilweise literaturbekannt oder können nach an sich literaturbekannten Verfahren (siehe Beispiele I bis V) oder den vorstehend beschriebenen Verfahren, gegebenenfalls unter zusätzlicher Einführung von Schutzresten erhalten werden.

Standardverfahren zur Herstellung der Ausgangsmaterialien sind beispielsweise in "March's Advanced Organic Chemistry" von Michael B. Smith and Jerry March, Wiley-VCH oder in "Science of Synthesis/Houben-Weyl", Thieme, beschrieben.

Eine beispielhafte Möglichkeit, Verbindungen der allgemeinen Formel (V) und (VI) zu erhalten, ist wie folgt:

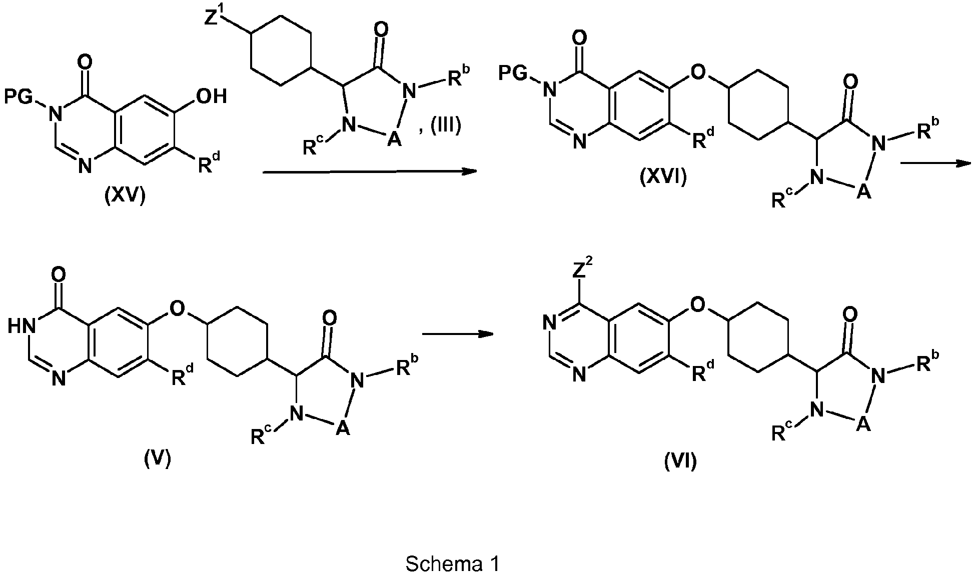

Ausgehend von einer Verbindung der allgemeinen Formel (XV), in der PG eine Schutzgruppe wie beispielsweise Benzyl, 4-Methoxybenzyl oder 2,4-Dimethoxybenzyl bedeutet, erfolgt die Umsetzung mit einer Verbindung der allgemeinen Formel (III) analog des vorstehend beschriebenen Verfahrens a) zu einer Verbindung der allgemeinen Formel (XVI). Die Verbindungen der allgemeinen Formel (XV) sind literaturbekannt (siehe z. B. WO 2004/108664 oder WO 2007/003486) oder können nach an sich literaturbekannten Verfahren erhalten werden.

Die Abspaltung des Schutzrestes aus einer Verbindung der allgemeinen Formel (XVI) zu einer Verbindung der allgemeinen Formel (V) erfolgt, falls PG Benzyl bedeutet, beispielsweise mit Wasserstoff in Gegenwart eines Katalysators wie Palladium/Kohle. Die Abspaltung des Schutzrestes, falls PG 4-Methoxybenzyl oder 2,4-Dimethoxybenzyl bedeutet, kann auch oxidativ (z.B. mit Cer(IV)-ammoniumnitrat oder mit 2,3-Dichlor-5,6-dicyano-1,4-benzochinon) oder mit Säuren (z.B. mit Trifluoressigssäure in Gegenwart von Anisol, Thioanisol, Pentamethylbenzol oder Triethylsilan) erfolgen.

Eine Verbindung der allgemeinen Formel (V) kann dann, wie im vorstehenden Verfahren c) beschrieben, in eine Verbindung der allgemeinen Formel (VI) übergeführt werden. Die Bedeutungen für **R^{b}, R^{c}, R^{d}, A,** Z¹ und Z² in den Verbindungen des Schema 1 sind wie vorstehend erwähnt definiert.

Eine weitere beispielhafte Möglichkeit, Verbindungen der allgemeinen Formel (XVI) zu erhalten, ist wie folgt:

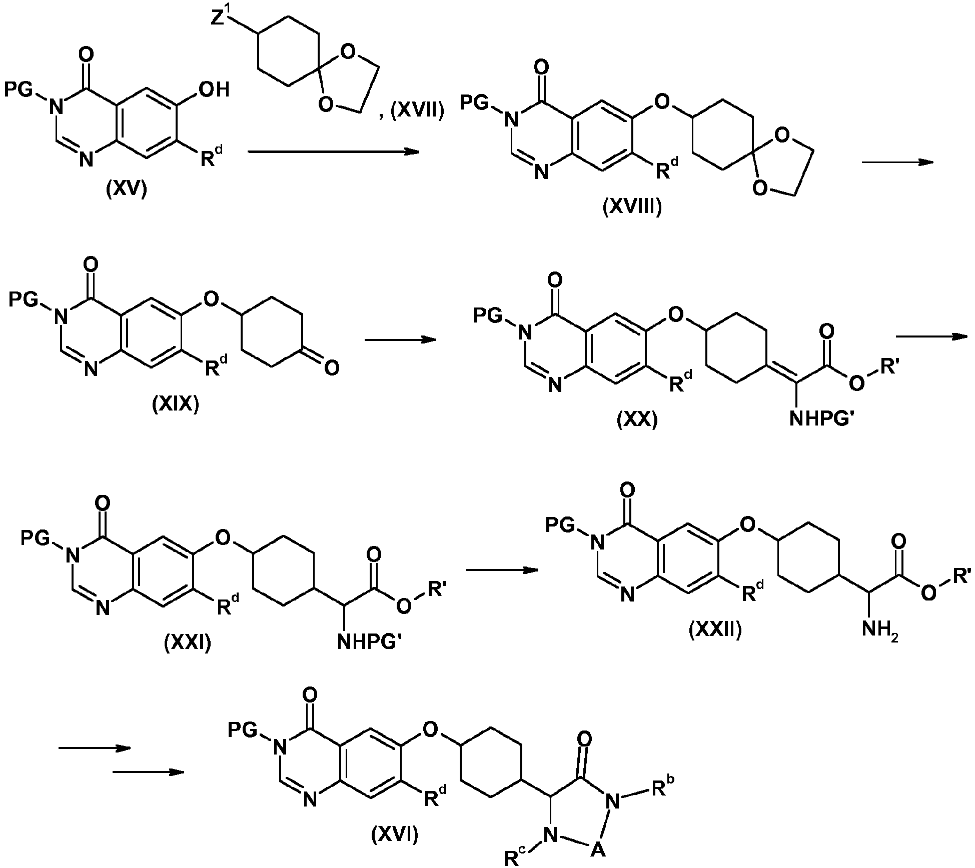

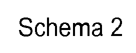

Ausgehend von einer Verbindung der allgemeinen Formel (XV), in der PG eine Schutzgruppe wie beispielsweise Benzyl, 4-Methoxybenzyl oder 2,4-Dimethoxybenzyl bedeutet, erfolgt die Umsetzung mit einer Verbindung der allgemeinen Formel (XVII) analog des vorstehend beschriebenen Verfahrens a) zu einer Verbindung der allgemeinen Formel (XVIII). Nach Spaltung des Ketals zum Keton erfolgt die Umsetzung zu einer Verbindung der allgemeinen Formel (XX) (analog z.B. WO 2008/079735). Nach Reduktion der Doppelbindung (analog z.B. WO 2008/079735) erfolgt Abspaltung des Schutzrestes PG' zu einer Verbindung der allgemeinen Formel (XXII). Die weiteren Schritte zu einer Verbindung der allgemeinen Formel (XVI) erfolgen beispielsweise analog zu den Beispielen III, II, I, und 1. Die Bedeutungen für **R^{b}, R^{c}, R^{d}, A** und Z¹ in den Verbindungen des Schema 2 sind wie vorstehend erwähnt definiert. PG' bedeutet eine Schutzgruppe, beispielsweise Formyl, Acetyl, Trifluoracetyl, Ethoxycarbonyl, tert.-Butoxycarbonyl oder Benzyloxycarbonyl und R' bedeutet eine C₁₋₄-Alkyl-Gruppe, beispielsweise Methyl oder Ethyl.

Eine weitere beispielhafte Möglichkeit, Verbindungen der allgemeinen Formel (XXI) zu erhalten, ist wie folgt:

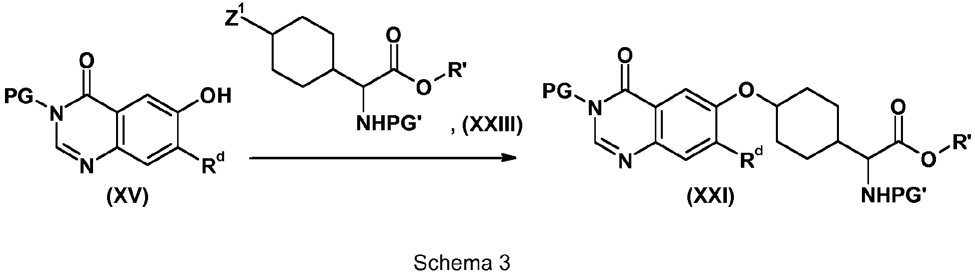

Ausgehend von einer Verbindung der allgemeinen Formel (XV), in der PG eine Schutzgruppe wie beispielsweise Benzyl, 4-Methoxybenzyl oder 2,4-Dimethoxybenzyl bedeutet, erfolgt die Umsetzung mit einer Verbindung der allgemeinen Formel (XXIII) analog des vorstehend beschriebenen Verfahrens a) zu einer Verbindung der allgemeinen Formel (XXI).

Die Bedeutungen für **R^{d},** PG, PG', R' und Z¹ in den Verbindungen des Schema 3 sind wie vorstehend erwähnt definiert.

Wie bereits eingangs erwähnt, weisen die erfindungsgemäßen Verbindungen der allgemeinen Formel (I) und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, dass die Signalübertragung an weiter abwärtsliegenden Komponenten blockiert wird.

Die nachfolgenden Beispiele sollen die vorliegende Erfindung näher erläutern ohne diese zu beschränken:

### Herstellung der Ausgangsverbindungen

### Beispiel I

### cis/trans-(S)-[(2-Amino-ethyl)-methyl-amino]-{4-[4-(3-chlor-2-fluor-phenylamino)-7 methoxy-chinazolin-6-yloxy]-cyclohexyl}-essigsäure-methylester

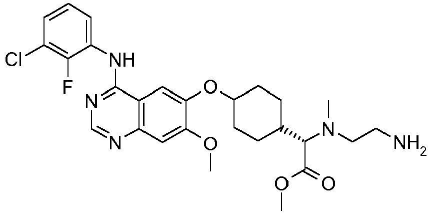

Ein Gemisch aus 970 mg cis/trans-(S)-[(2-tert.-Butoxycarbonylamino-ethyl)-methyl-amino]-{4-[4-(3-chlor-2-fluor-phenylamino)-7-methoxy-chinazolin-6-yloxy]-cyclohexyl}-essigsäure-methylester, 2 ml Trifluoressigsäure und 8 ml Methylenchlorid wird über Nacht bei Raumtemperatur gerührt. Anschließend wird Reaktionsgemisch im Vakuum eingeengt, wobei ein bräunlicher Feststoff zurückbleibt, welcher ohne weitere Reinigung weiter umgesetzt wird.
Ausbeute: 700 mg (85 % der Theorie)
Massenspektrum (ESI⁺): m/z = 546, 548 [M+H]⁺

Analog Beispiel I werden folgende Verbindungen erhalten:
(1) cis/trans-(S)-Amino-{4-[4-(3-chlor-2-fluor-phenylamino)-7-methoxy-chinazolin-6-yloxy]-cyclohexyl}-essigsäure-methylester 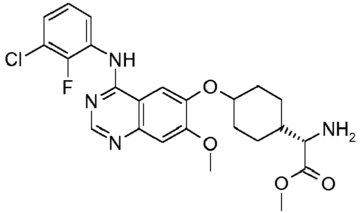 R_{f}-Wert: 0.42 (Kieselgel, Methylenchlorid/Methanol/konz. Ammoniak = 90:10:1)
   Massenspektrum (ESI⁺): m/z = 489, 491 [M+H]⁺
(2) cis/trans-(S)-(2-Amino-ethylamino)-{4-[4-(3-chlor-2-fluor-phenylamino)-7-methoxy-chinazolin-6-yloxy]-cyclohexyl}-essigsäure-methylester 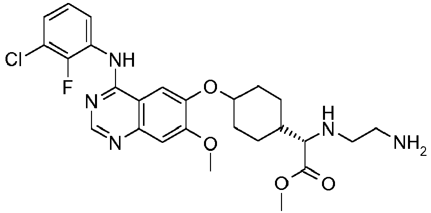 Massenspektrum (ESI⁺): m/z = 532, 534 [M+H]⁺
(3) cis/trans-(S)-{4-[4-(3-Chlor-2-fluor-phenylamino)-7-methoxy-chinazolin-6-yloxy]-cyclohexyl}-(2-methylamino-ethylamino)-essigsäure-methylester 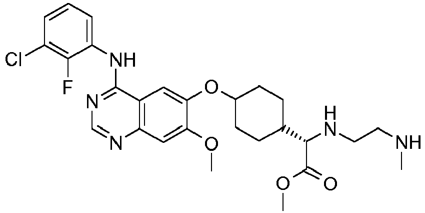 Massenspektrum (ESI⁺): m/z = 546, 548 [M+H]⁺
(4) cis/trans-(S)-{4-[4-(3-Chlor-2-fluor-phenylamino)-7-methoxy-chinazolin-6-yloxy]-cyclohexyl}-[methyl-(2-methylamino-ethyl)-amino]-essigsäure-methylester 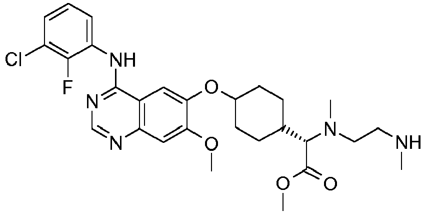 Massenspektrum (ESI⁺): m/z = 560, 562 [M+H]⁺
(5) cis-(R)-(2-Amino-ethylamino)-{4-[4-(3-chlor-2-fluor-phenylamino)-7-methoxy-chinazolin-6-yloxy]-cyclohexyl}-essigsäure-methylester 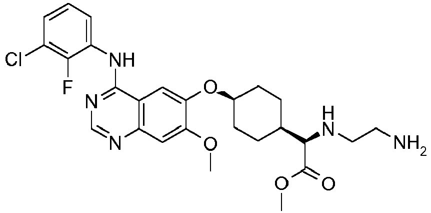 Massenspektrum (ESI⁻): m/z = 530, 532 [M+H]⁻
(6) cis-(R)-Amino-{4-[4-(3-chlor-2-fluor-phenylamino)-7-methoxy-chinazolin-6-yloxy]-cyclohexyl}-essigsäure-methylester 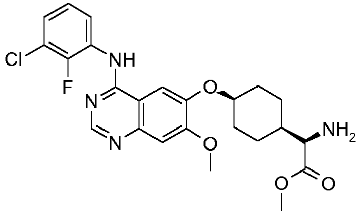 Massenspektrum (ESI⁺): m/z = 489, 491 [M+H]⁺
(7) trans-(R)-(2-Amino-ethylamino)-{4-[4-(3-chlor-2-fluor-phenylamino)-7-methoxy-chinazolin-6-yloxy]-cyclohexyl}-essigsäure-methylester 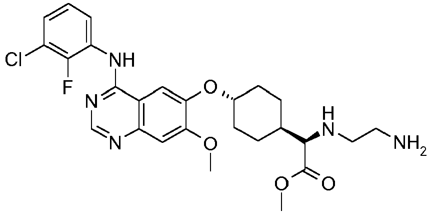 Massenspektrum (ESI⁺): m/z = 532, 534 [M+H]⁺
(8) trans-(R)-Amino-{4-[4-(3-chlor-2-fluor-phenylamino)-7-methoxy-chinazolin-6-yloxy]-cyclohexyl}-essigsäure-methylester 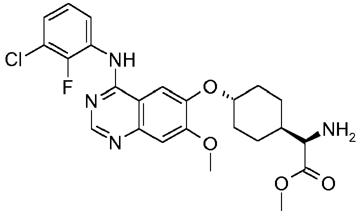 Massenspektrum (ESI⁺): m/z = 489, 491 [M+H]⁺

### Beispiel II

### cis/trans-(S)-[(2-tert.-Butoxycarbonylamino-ethyl)-methyl-amino]-{4-[4-(3-chlor-2-fluor-phenylamino)-7-methoxy-chinazolin-6-yloxy]-cyclohexyl}-essigsäure-methylester

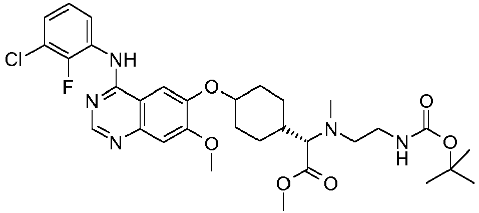

Zu 1.00 g cis/trans-(S)-(2-tert.-Butoxycarbonylamino-ethylamino)-{4-[4-(3-chlor-2-fluor-phenylamino)-7-methoxy-chinazolin-6-yloxy]-cyclohexyl}-essigsäure-methylester in 30 ml Tetrahydrofuran werden 240 µl einer 37 %igen wässrigen Formaldehyd-Lösung, 100 µl Eisessig und 500 mg Natriumtriacetoxyborhydrid gegeben. Das Reaktionsgemisch wird über Nacht bei Raumtemperatur gerührt, mit Essigester verdünnt, mit 10%iger Kaliumcarbonat-Lösung versetzt und kräftig gerührt. Die organische Phase wird abgetrennt, mit Wasser und gesättigter Natriumchlorid-Lösung gewaschen, über Magnesiumsulfat getrocknet und eingeengt.
Ausbeute: 970 mg (95 % der Theorie)
Massenspektrum (ESI⁺): m/z = 646, 648 [M+H]⁺

Analog Beispiel II wird folgende Verbindung erhalten:
(1) cis/trans-(S)-{[2-(tert.-Butoxycarbonyl-methyl-amino)-ethyl]-methyl-amino}-{4-[4-(3-chlor-2-fluor-phenylamino)-7-methoxy-chazolin-6-yloxy]-cyclohexyl}-essigsäure-methylester 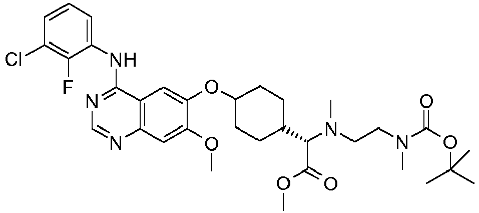 Massenspektrum (ESI⁺): m/z = 660, 662 [M+H]⁺

### Beispiel III

### cis/trans-(S)-(2-tert.-Butoxycarbonylamino-ethylamino)-{4-[4-(3-chlor-2-fluor-phenylamino)-7-methoxy-chinazolin-6-yloxy]-cyclohexyl}-essigsäure-methylester

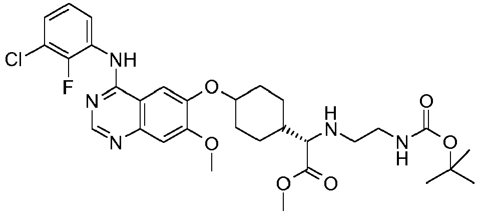

Zu 1.50 g cis/trans-(S)-Amino-{4-[4-(3-chlor-2-fluor-phenylamino)-7-methoxy-chinazolin-6-yloxy]-cyclohexyl}-essigsäure-methylester in 40 ml Tetrahydrofuran werden 490 mg (2-Oxo-ethyl)-carbaminsäure-tert.-butylester gefolgt von 180 µl Eisessig und 150 mg Natriumtriacetoxyborhydrid gegeben.

Das Reaktionsgemisch wird über Nacht bei Raumtemperatur gerührt, dann werden nochmals 70 mg (2-Oxo-ethyl)-carbaminsäure-tert.-butylester und 150 mg Natriumtriacetoxyborhydrid zugegeben und weitere vier Stunden bei Raumtemperatur gerührt. Zu Aufarbeitung wird das Reaktionsgemisch mit 20 ml Essigester verdünnt, mit 10 ml 10%iger Kaliumcarbonat-Lösung versetzt und gründlich verrührt. Die wässrige Phase wird abgetrennt und mit Essigester extrahiert. Die vereinigten organischen Phasen werden mit gesättigter Natriumchlorid-Lösung gewaschen, über Magnesiumsulfat getrocknet und eingeengt. Das Rohprodukt wird ohne weitere Reinigung weiter umgesetzt.
Massenspektrum (ESI⁺): m/z = 632, 634 [M+H]⁺

Analog Beispiel III werden folgende Verbindungen erhalten:
(1) cis/trans-(S)-[2-(tert.-Butoxycarbonyl-methyl-amino)-ethylamino]-{4-[4-(3-chlor-2-fluor-phenylamino)-7-methoxy-chinazolin-6-yloxy]-cyclohexyl}-essigsäure-methylester 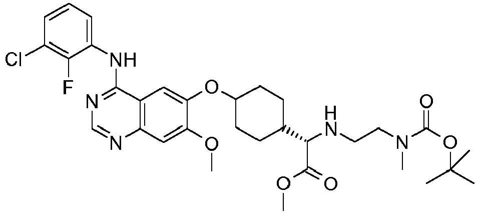
   R_{f}-Wert: 0.16 (Kieselgel, Methylenchlorid/Methanol/konz. Ammoniak = 95:5:0.1)
   Massenspektrum (ESI⁺): m/z = 646, 648 [M+H]⁺
(2) cis-(R)-(2-tert.-Butoxycarbonylamino-ethylamino)-{4-[4-(3-chlor-2-fluor-phenylamino)-7-methoxy-chinazolin-6-yloxy]-cyclohexyl}-essigsäure-methylester 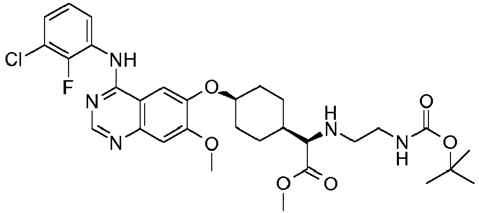 Massenspektrum (ESI⁺): m/z = 632, 634 [M+H]⁺
(3) trans-(R)-(2-tert.-Butoxycarbonylamino-ethylamino)-{4-[4-(3-chlor-2-fluor-phenylamino)-7-methoxy-chinazolin-6-yloxy]-cyclohexyl}-essigsäure-methylester 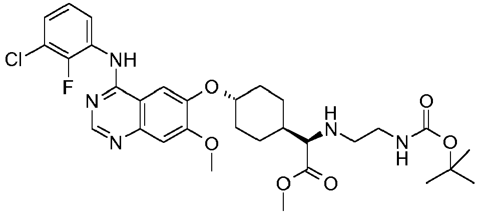
Massenspektrum (ESI⁺): m/z = 632, 634 [M+H]⁺

### Beispiel IV

### cis/trans-(S)-tert.-Butoxycarbonylamino-{4-[4-(3-chlor-2-fluor-phenylamino)-7-methoxy-chinazolin-6-yloxy]-cyclohexyl}-essigsäure-methylester

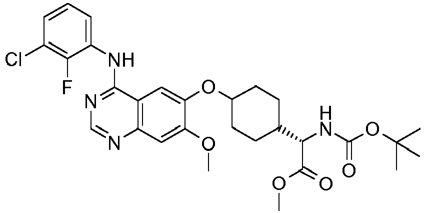

10.00 g 4-(3-Chlor-2-fluor-phenylamino)-7-methoxy-chinazolin-6-ol in 70 ml N,N-Dimethylformamid werden auf 50 °C erwärmt, mit 7.00 g Kaliumcarbonat sowie 14.00 g cis/trans-(S)-tert.-Butoxycarbonylamino-(4-methansulfonyloxy-cyclohexyl)-essigsäure-methylester versetzt und auf 80 °C erhitzt. Das Reaktionsgemisch wird über Nacht bei 80 °C gerührt, dann werden nochmals 3.50 g cis/trans-(S)-tert.-Butoxycarbonylamino-(4-methansulfonyloxy-cyclohexyl)-essigsäure-methylester und 2.60 g Kaliumcarbonat zugegeben.

Nach weiteren vier Stunden bei 80 °C wird das Reaktionsgemisch abgekühlt und mit Essigester und Wasser verdünnt. Die wässrige Phase wird abgetrennt und mit Essigester extrahiert. Die vereinigten organischen Phasen werden über Magnesiumsulfat getrocknet und eingeengt. Das Rohprodukt wird chromatographisch über eine Kieselgelsäule mit Methylenchlorid/Methanol/konz. Ammoniak (99/1/0.1 auf 8/2/0.1) als Laufmittel gereinigt.
Ausbeute: 5.90 g (32 % der Theorie)
Massenspektrum (ESI⁺): m/z = 589, 591 [M+H]⁺

Analog Beispiel IV werden folgende Verbindungen erhalten:
(1) cis-(R)-tert.-Butoxycarbonylamino-{4-[4-(3-chlor-2-fluor-phenylamino)-7-methoxy-chinazolin-6-yloxy]-cyclohexyl}-essigsäure-methylester 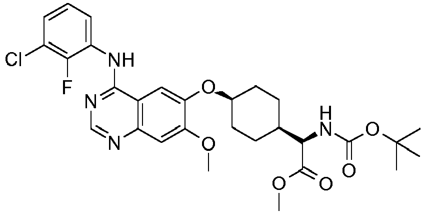 Massenspektrum (ESI⁺): m/z = 589, 591 [M+H]⁺
(2) trans-(R)-tert.-Butoxycarbonylamino-{4-[4-(3-chlor-2-fluor-phenylamino)-7-methoxy-chinazolin-6-yloxy]-cyclohexyl}-essigsäure-methylester 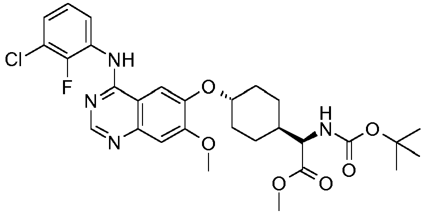 Massenspektrum (ESI⁺): m/z = 589, 591 [M+H]⁺

### Beispiel V

### cis/trans-(S)-tert.-Butoxycarbonylamino-(4-methansulfonyloxy-cyclohexyl)-essigsäure-methylester

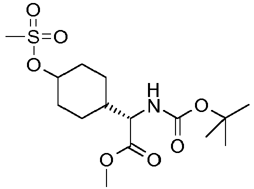

Zu 17.55 g cis/trans-(S)-tert.-Butoxycarbonylamino-(4-hydroxy-cyclohexyl)-essigsäure-methylester (s. WO2004/110436) und 10.50 ml Triethylamin in 170 ml Methylenchlorid werden unter Eisbad-Kühlung langsam 5.20 ml Methansulfonsäurechlorid getropft, wobei Temperatur unter 10°C gehalten wird. Anschließend wird das Reaktionsgemisch auf Raumtemperatur erwärmt und über Nacht gerührt.

Zur Aufarbeitung werden 50 ml gesättigte Natriumhydrogencarbonat-Lösung zugegeben. Die wässrige Phase wird abgetrennt und mit Methylenchlorid extrahiert. Die vereinigten organischen Phasen werden mit gesättigter Natriumchlorid-Lösung gewaschen, über Magnesiumsulfat getrocknet und eingeengt. Es bleibt ein zähes Öl zurück, welches ohne weitere Reinigung weiter umgesetzt wird.
Ausbeute: 21.70 g (97 % der Theorie)
Massenspektrum (ESI⁺): m/z = 383 [M+NH₄]⁺

Analog Beispiel V werden folgende Verbindungen erhalten:
(1) cis-(R)-tert.-Butoxycarbonylamino-(4-methansulfonyloxy-cyclohexyl)-essigsäure-methylester 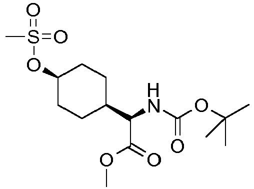 Das Ausgangsmaterial, cis-(R)-tert.-Butoxycarbonylamino-(4-hydroxy-cyclohexyl)-essigsäure-methylester, wird ausgehend von (R)-4-Hydroxy-phenylglycin analog WO2004/110436 erhalten.
   Massenspektrum (ESI⁺): m/z = 366 [M+H]⁺
(2) trans-(R)-tert.-Butoxycarbonylamino-(4-methansulfonyloxy-cyclohexyl)-essigsäure-methylester 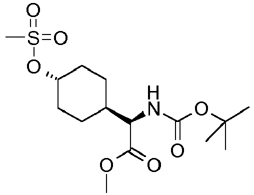 Das Ausgangsmaterial, trans-(R)-tert.-Butoxycarbonylamino-(4-hydroxy-cyclohexyl)-essigsäure-methylester, wird ausgehend von (R)-4-Hydroxy-phenylglycin analog WO2004/110436 erhalten.
   Massenspektrum (ESI⁺): m/z = 366 [M+H]⁺

### Herstellung der Endverbindungen

### Beispiel 1

### cis/trans-(S)-3-{4-[4-(3-Chlor-2-fluor-phenylamino)-7-methoxy-chinazolin-6-yloxy]-cyclohexyl}-4-methyl-piperazin-2-on

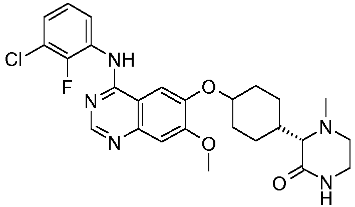

Ein Gemisch aus 700 mg [(2-Amino-ethyl)-methyl-amino]-{4-[4-(3-chlor-2-fluor-phenylamino)-7-methoxy-chinazolin-6-yloxy]-cyclohexyl}-essigsäure-methylester, Methanol 7 ml und 0.65 ml 4N Natronlauge wird drei Stunden bei Raumtemperatur gerührt. Anschließend wird das Reaktionsgemisch eingeengt und mit Essigester extrahiert. Die organische Phase wird über Magnesiumsulfat getrocknet und eingeengt. Der Kolbenrückstand wird chromatographisch über eine Kieselgelsäule mit Methylenchlorid/Methanol/konz. Ammoniak (98/2/0.1 auf 8/2/0.1) als Laufmittel gereinigt. Das Rohprodukt wird mit Diisopropylether verrührt, abgesaugt und getrocknet.
Ausbeute: 300 mg (46 % der Theorie)
Massenspektrum (ESI⁺): m/z = 514, 516 [M+H]⁺

Analog Beispiel 1 werden folgende Verbindungen erhalten:
(1) cis/trans-(S)-3-{4-[4-(3-Chlor-2-fluor-phenylamino)-7-methoxy-chinazolin-6-yloxy]-cyclohexyl}-piperazin-2-on 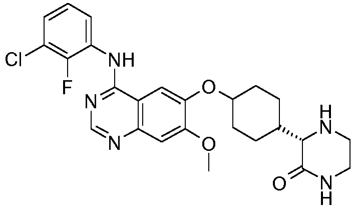 R_{f}-Wert: 0.20 (Kieselgel, Methylenchlorid/Methanol/konz. Ammoniak = 90:10:1)
   Massenspektrum (ESI⁺): m/z = 500, 502 [M+H]⁺
   Das cis/trans-Gemisch wird durch überkritische Flüssigkeitschromatographie unter den folgenden Bedingungen getrennt:
   Säule: Daicel ASH 20x250 mm
   Eluent: CO₂/Methanol (+ 0.2% Diethylamin) (60:40)
   Fluss: 70 ml/min
   Injektionsvolumen: 450 µl
   Probenkonzentration: 100mg/ml

   Die Zuordnung der Isomeren erfolgt über 1H-NMR Spektroskopie (400MHz, Dimethylsulfoxid-d6)
   a) cis-(S)-3-{4-[4-(3-Chlor-2-fluor-phenylamino)-7-methoxy-chinazolin-6-yloxy]-cyclohexyl}-piperazin-2-on 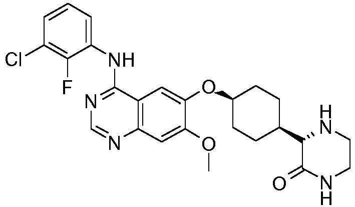 charakteristisches Signal bei 4.75 (1 H, m)
   b) trans-(S)-3-{4-[4-(3-Chlor-2-fluor-phenylamino)-7-methoxy-chinazolin-6-yloxy]-cyclohexyl}-piperazin-2-on 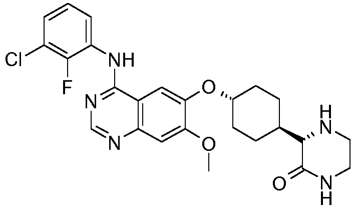 charakteristisches Signal bei 4.42 (1 H, m)
(2) trans-(S)-3-{4-[4-(3-Chlor-2-fluor-phenylamino)-7-methoxy-chinazolin-6-yloxy]-cyclohexyl}-1-methyl-piperazin-2-on 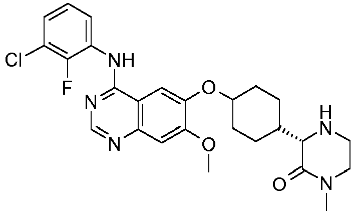 R_{f}-Wert: 0.31 (Kieselgel, Methylenchlorid/Methanol/konz. Ammoniak = 90:10:1)
   Massenspektrum (ESI⁺): m/z = 514, 516 [M+H]⁺
(3) cis/trans-(S)-3-{4-[4-(3-Chlor-2-fluor-phenylamino)-7-methoxy-chinazolin-6-yloxy]-cyclohexyl}-1,4-dimethyl-piperazin-2-on 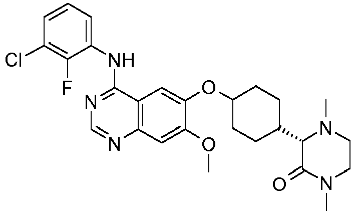 R_{f}-Wert: 0.35 (Kieselgel, Methylenchlorid/Methanol/konz. Ammoniak = 90:10:1)
   Massenspektrum (ESI⁺): m/z = 528, 530 [M+H]⁺
(4) cis-(R)-3-{4-[4-(3-Chlor-2-fluor-phenylamino)-7-methoxy-chinazolin-6-yloxy]-cyclohexyl}-piperazin-2-on 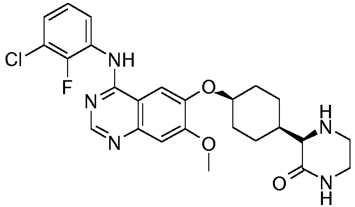 Die Cyclisierung wird in Methanol in Gegenwart von Triethylamin bei Raumtemperatur durchgeführt.
   Massenspektrum (ESI⁺): m/z = 500, 502 [M+H]⁺
(5) trans-(R)-3-{4-[4-(3-Chlor-2-fluor-phenylamino)-7-methoxy-chinazolin-6-yloxy]-cyclohexyl}-piperazin-2-on 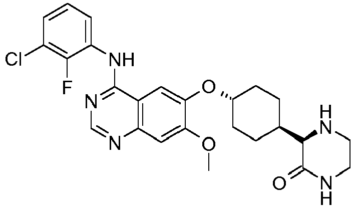 Die Cyclisierung wird in Methanol in Gegenwart von Triethylamin bei Raumtemperatur durchgeführt.
   Massenspektrum (ESI⁺): m/z = 500, 502 [M+H]⁺

### Biologischer Test

Die biologischen Eigenschaften der neuen Verbindungen werden beispielsweise wie folgt geprüft:
Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Eine murine hämatopoetische Zelllinie wird derart genetisch verändert, dass sie funktionellen humanen EGF-R exprimiert. Die Proliferation dieser Zelllinie kann daher durch EGF stimuliert werden.

Der Test wird wie folgt durchgeführt:
Die Zellen werden in RPMI/1640 Medium kultiviert. Die Proliferation wird mit 20 ng/ml humanem EGF (Promega) stimuliert. Zur Untersuchung der inhibitorischen Aktivität der erfindungsgemäßen Verbindungen werden diese Verbindungen in 100% Dimethylsulfoxid (DMSO) gelöst und in verschiedenen Verdünnungen den Kulturen zugefügt, wobei die maximale DMSO Konzentration 1 % beträgt. Die Kulturen werden für 48 Stunden bei 37°C inkubiert.

Zur Bestimmung der inhibitorischen Aktivität der erfindungsgemäßen Verbindungen wird die relative Zellzahl mit dem Cell Titer 96TM AQueous Non-Radioactive Cell Proliferation Assay (Promega) in O.D. Einheiten gemessen. Die relative Zellzahl wird in Prozent der Kontrolle berechnet und die Wirkstoffkonzentration, die die Proliferation der Zellen zu 50% hemmt (IC50), abgeleitet.

Die erfindungsgemäßen Verbindungen der allgemeinen Formel (I) zeigen beispielsweise IC50-Werte von < 10 micromolar, vorzugsweise von < 1 micromolar.

| Verbindung (Beispiel Nr.) | Hemmung der EGFR-abhängigen Proliferation IC₅₀ [nM] |
|---|---|
| 1 | 1.0 |
| 1(1a) | 10 |
| 1(1b) | 1.4 |
| 1(2) | 1.0 |
| 1(3) | 1.0 |
| 1(4) | 12.5 |
| 1(5) | 1.6 |

### Indikationsgebiete

Wie gefunden wurde, zeichnen sich die Verbindungen der Formel **(I)** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **(I)** aufgrund ihrer pharmazeutischen Wirksamkeit als Tyrosinkinase-Hemmer bevorzugt zur Anwendung gelangen können.

Die erfindungsgemäßen Verbindungen der allgemeinen Formel (I) hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuro-epithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Menetrier, sezernierende Adenome und Proteinverlustsyndrome.

Außerdem können die Verbindungen der allgemeinen Formel (I) und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), benigner Prostatahyperplasie (BPH), inflammatorischer Prozesse, Erkrankungen des Immunsystems, Hyperproliferation hämatopoetischer Zellen, der Behandlung von Nasenpolypen, etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch (z.B. Ambroxol, N-acetylcystein), broncholytisch (z.B. Tiotropium oder Ipratropium oder Fenoterol, Salmeterol, Salbutamol) und/oder entzündungshemmend (z.B. Theophylline oder Glucocorticoide) wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden Substanzen gegeben werden. Diese Kombinationen können entweder simultan oder sequentiell verabreicht werden.

### Formulierungen

Die erfindungsgemäßen Verbindungen können oral, transdermal, inhalativ, parenteral oder sublingual verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragees, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 0.1 und 5000, vorzugsweise zwischen 1 und 500, besonders bevorzugt zwischen 5-300 mg/Dosis, bei intravenöser, subkutaner oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern, ethanolischen oder wässrigen Lösungen bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Bei der pharmazeutischen Anwendung werden die erfindungsgemäßen Verbindungen in der Regel bei warmblütigen Wirbeltieren, insbesondere beim Menschen, in Dosierungen von 0.01-100 mg/kg Körpergewicht, vorzugsweise bei 0.1-15 mg/kg verwendet. Zur Verabreichung können diese beispielsweise mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet werden.

Die nachfolgenden Beispiele illustrieren die vorliegende Erfindung ohne sie jedoch in ihrem Umfang zu beschränken:

### Pharmazeutische Formulierungsbeispiele

### A) Dragees mit 75 mg Wirksubstanz

### Zusammensetzung:

**1 Drageekern enthält:**

| | |
|---|---|
| Wirksubstanz | 75.0 mg |
| Calciumphosphat | 93.0 mg |
| Maisstärke | 35.5 mg |
| Polyvinylpyrrolidon | 10.0 mg |
| Hydroxypropylmethylcellulose | 15.0 mg |
| Magnesiumstearat | 1.5 mg |
| | 230.0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Drageekerne werden mit einem Film überzogen, der im wesentlichen aus Hydroxypropylmethylcellulose besteht. Die fertigen Filmdragees werden mit Bienenwachs geglänzt.

| | |
|---|---|
| Drageegewicht: | 245 mg. |

### B) Tabletten mit 100 mg Wirksubstanz

### Zusammensetzung:

**1 Tablette enthält:**

| | |
|---|---|
| Wirksubstanz | 100.0 mg |
| Milchzucker | 80.0 mg |
| Maisstärke | 34.0 mg |
| Polyvinylpyrrolidon | 4.0 mg |
| Magnesiumstearat | 2.0 mg |
| | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | | |
|---|---|---|
| Tablettengewicht: | 220 mg | |
| Durchmesser: | 10 mm, | biplan mit beidseitiger Facette und einseitiger Teilkerbe. |

### C) Tabletten mit 150 mg Wirksubstanz

### Zusammensetzung:

**1 Tablette enthält:**

| | |
|---|---|
| Wirksubstanz | 150.0 mg |
| Milchzucker pulv. | 89.0 mg |
| Maisstärke | 40.0 mg |
| Kolloide Kieselgelsäure | 10.0 mg |
| Polyvinylpyrrolidon | 10.0 mg |
| Magnesiumstearat | 1.0 mg |
| | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### D) Hartgelatine-Kapseln mit 150 mg Wirksubstanz

### Zusammensetzung:

**1 Kapsel enthält:**

| | | |
|---|---|---|
| Wirkstoff | | 150.0 mg |
| Maisstärke getr. | ca. | 180.0 mg |
| Milchzucker pulv. | ca. | 87.0 mg |
| Magnesiumstearat | | 3.0 mg |
| ca. | | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.

Die Endmischung wird in Hartgelatine-Kapseln der Größe 1 abgefüllt.
Kapselfüllung: ca. 320 mg
Kapselhülle: Hartgelatine-Kapsel Größe 1.

### E) Suppositorien mit 150 mg Wirksubstanz

### Zusammensetzung:

**1 Zäpfchen enthält:**

| | |
|---|---|
| Wirkstoff | 150.0 mg |
| Polyäthylenglykol 1500 | 550.0 mg |
| Polyäthylenglykol 6000 | 460.0 mg |
| Polyoxyäthylensorbitanmonostearat | 840.0 mg |
| | 2000.0 mg |

### Herstellung:

Nach dem Aufschmelzen der Suppositorienmasse wird der Wirkstoff darin homogen verteilt und die Schmelze in vorgekühlte Formen gegossen.

### F) Suspension mit 50 mg Wirksubstanz

### Zusammensetzung:

**100 ml Suspension enthalten:**

| | |
|---|---|
| Wirkstoff | 1.00 g |
| Carboxymethylcellulose-Na-Salz | 0.10 g |
| p-Hydroxybenzoesäuremethylester | 0.05 g |
| p-Hydroxybenzoesäurepropylester | 0.01 g |
| Rohrzucker | 10.00 g |
| Glycerin | 5.00 g |
| Sorbitlösung 70%ig | 20.00 g |
| Aroma | 0.30 g |
| Wasser dest. ad | 100.00 ml |

### Herstellung:

Destilliertes Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### G) Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### H) Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

### l) Kapseln zur Pulverinhalation mit 5 mg Wirksubstanz

**1 Kapsel enthält:**

| | |
|---|---|
| Wirksubstanz | 5.0 mg |
| Lactose für Inhalationszwecke | 15.0 mg |
| | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70.0 mg
Kapselgröße: 3

### J) Inhalationslösung für Handvernebler mit 2.5 mg Wirksubstanz

**1 Hub enthält:**

| | |
|---|---|
| Wirksubstanz | 2.500 mg |
| Benzalkoniumchlorid | 0.001 mg |
| 1N-Salzsäure q.s. | |
| Ethanol/Wasser (50/50) | ad 15.000 mg |

### Herstellung:

Die Wirksubstanz und Benzalkoniumchlorid werden in Ethanol/Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.
Füllmasse des Behälters: 4.5 g

## Patentansprüche

1. Verbindungen der allgemeinen Formel (I) 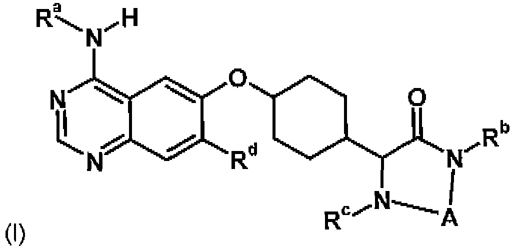 **dadurch gekennzeichnet, dass**
**R^{a}** 3-Chlor-2-fluor-phenyl-
**R^{b}** Wasserstoff, oder einen Rest, gegebenenfalls substituiert, ausgewählt aus der Gruppe bestehend aus C₁₋₆-Alkyl, C₃₋₆-Cycloalkyl- und C₃₋₆-Cycloalkyl-C₁₋₃-alkyl,
**R^{c}** Wasserstoff, oder einen Rest, gegebenenfalls substituiert, ausgewählt aus der Gruppe bestehend aus C₁₋₆-Alkyl, C₃₋₆-Cycloalkyl-, C₃₋₆-Cycloalkyl-C₁₋₃-alkyl, C₁₋₆-Alkyl-CO-, C₃₋₆-Cycloalkyl-CO-, C₃₋₆-Cycloalkyl-C₁₋₃-alkyl-CO-, C₁₋₆-Alkyl-SO₂-, C₃₋₈-Cycloalkyl-SO₂-, C₃₋₆-Cycloalkyl-C₁₋₃-alkyl-SO₂-, Phenyl-CO- und Phenyl-SO₂-,
**R^{d}** -O-Me
**A** -CH₂-CH₂-
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

2. Verbindungen nach Anspruch 1,
**dadurch gekennzeichnet, dass**
**R^{b}** und **R^{c}** gleich oder verschieden, Wasserstoff oder C₁₋₃-Alkyl,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

3. Verbindungen der Formel (I) nach einem der Ansprüche 1 bis 2 zur Verwendung als Arzneimittel.

4. Verbindungen der Formel (I) nach Anspruch 3 zur Anwendung in der Behandlung entzündlicher oder allergischer Erkrankungen der Atemwege.

5. Verbindungen der Formel (I) nach Anspruch 3 zur Anwendung in der Behandlung einer Erkrankung, die ausgewählt ist aus der Gruppe bestehend aus Chronischer Bronchitis, akuter Bronchitis, Bronchitis aufgrund bakterieller oder viraler Infektion oder Pilzen oder Helminthen, allergischer Bronchitis, toxischer Bronchitis, chronisch obstruktiver Bronchitis (COPD), Asthma (intrinsisch oder allergisch), pediatrischem Asthma, Bronchiektasien, allergischer Alveolitis, allergischer oder nicht-allergischer Rhinitis, chronischer Sinusitis, zystischer Fibrose oder Mukoviszidose, alpha-1-Antitrypsin-Mangel, Husten, Lungenemphysem, interstitieller Lungenerkrankungen, Alveolitis, hyperreaktiver Atemwege, Nasenpolypen, Lungenödemen, Pneumonitis aufgrund unterschiedlicher Genese wie Strahlen-induziert oder durch Aspiration oder infektiöse, Kollagenosen wie Lupus eryth, systemische Sklerodermie, Sarkoidose und M. Boeck.

6. Verbindungen der Formel (I) nach Anspruch 3 zur Anwendung in der Behandlung entzündlicher oder allergischer Krankheitszustände bei denen Autoimmun-Reaktionen beteiligt sind.

7. Verbindungen der Formel (I) nach Anspruch 3 zur Anwendung in der Behandlung einer Erkrankung in Form von benignen oder malignen Tumoren.

8. Pharmazeutische Formulierung enthaltend eine Verbindung der Formel (I) gemäß einem der Ansprüche 1 oder 2.

9. Oral applizierbare pharmazeutische Formulierung nach Anspruch 8 enthaltend eine Verbindung der Formel (1) gemäß Anspruch 1 oder 2.

10. Arzneimittelkombinationen, die neben einer oder mehrerer Verbindungen der Formel (I) gemäß einem der Ansprüche 1 oder 2 als weiteren Wirkstoff einen oder mehrere Verbindungen enthalten, die ausgewählt sind aus den Klassen der Betamimetika, Anticholinergika, Corticosteroiden, weitere PDE4-Inhibitoren, LTD4-Antagonisten, EGFR-Hemmern, Dopamin-Agonisten, H1-Antihistaminika, PAF-Antagonisten und PI3-Kinase Inhibitoren oder zwei- oder dreifach Kombinationen davon.

## Claims

1. Compounds of general formula (I) 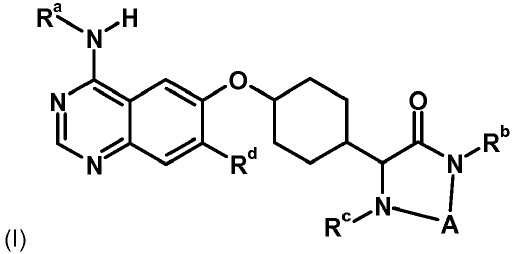 **characterised in that**
**R^{a}** denotes 3-chloro-2-fluoro-phenyl,
**R^{b}** denotes hydrogen, or an optionally substituted group selected from among C₁₋₆-alkyl, C₃₋₆-cycloalkyl and C₃₋₆-cycloalkyl-C₁₋₃-alkyl,
**R^{c}** denotes hydrogen, or an optionally substituted group selected from among C₁₋₆-alkyl, C₃₋₆-cycloalkyl, C₃₋₆-cycloalkyl-C₁₋₃-alkyl, C₁₋₆-alkyl-CO, C₃₋₆-cycloalkyl-CO, C₃₋₆-cycloalkyl-C₁₋₃-alkyl-CO, C₁₋₆-alkyl-SO₂, C₃₋₆-cycloalkyl-SO₂, C₃₋₆-cycloalkyl-C₁₋₃-alkyl-SO₂, phenyl-CO- and phenyl-SO₂,
**R^{d}** denotes -O-Me
**A** denotes -CH₂-CH₂-
optionally in the form of the tautomers, the racemates, the enantiomers, the diastereomers and the mixtures thereof, and optionally the pharmacologically acceptable acid addition salts thereof.

2. Compounds according to claim 1,
**characterised in that**
**R^{b}** and **R^{c}** which may be identical or different, denote hydrogen or C₁₋₃-alkyl,
optionally in the form of the tautomers, the racemates, the enantiomers, the diastereomers and the mixtures thereof, and optionally the pharmacologically acceptable acid addition salts thereof.

3. Compounds of formula (I) according to one of claims 1 to 2 for use as medicaments.

4. Compounds of formula (I) according to claim 3 for use in the treatment of inflammatory or allergic diseases of the airways.

5. Compounds of formula (I) according to claim 3 for use in the treatment of a disease selected from among chronic bronchitis, acute bronchitis, bronchitis caused by bacterial or viral infection or fungi or helminths, allergic bronchitis, toxic bronchitis, chronic obstructive bronchitis (COPD), asthma (intrinsic or allergic), paediatric asthma, bronchiectasis, allergic alveolitis, allergic or non-allergic rhinitis, chronic sinusitis, cystic fibrosis or mucoviscidosis, alpha-1-antitrypsin deficiency, cough, pulmonary emphysema, interstitial lung diseases, alveolitis, hyperreactive airways, nasal polyps, pulmonary oedema, pneumonitis of different origins, e.g. radiation-induced or caused by aspiration or infectious pneumonitis, collagenoses such as lupus erythematodes, systemic scleroderma, sarcoidosis and Boeck's disease.

6. Compounds of formula (I) according to claim 3 for use in the treatment of inflammatory or allergic conditions in which autoimmune reactions are involved.

7. Compounds of formula (I) according to claim 3 for use in the treatment of a disease in the form of benign or malignant tumours.

8. Pharmaceutical formulation containing a compound of formula (I) according to one of claims 1 or 2.

9. Orally administered pharmaceutical formulation according to claim 8 containing a compound of formula (I) according to claim 1 or 2.

10. Medicament combinations which contain, besides one or more compounds of formula (I) according to one of claims 1 or 2, as further active substances, one or more compounds selected from among the categories of betamimetics, anticholinergics, corticosteroids, further PDE4-inhibitors, LTD4-antagonists, EGFR-inhibitors, dopamine agonists, H1-antihistamines, PAF-antagonists and PI3-kinase inhibitors or double or triple combinations thereof.

## Revendications

1. Composés de formule générale (I) 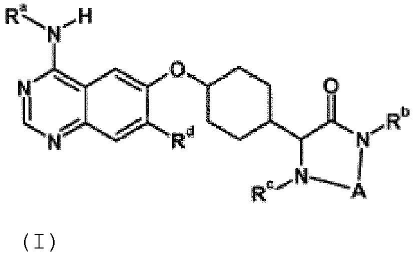 **caractérisés en ce que**
R^{a} représente 3-chlor-2-fluor-phényl-
R^{b} représente hydrogène, ou un résidu, éventuellement substitué, sélectionné parmi le groupe constitué de C₁₋₆-alkyle, C₃₋₆-cycloakyl- et C₃₋₆-cycloalkyl-C₁₋₃-alkyle,
R^{c} représente hydrogène, ou un résidu, éventuellement substitué, sélectionné parmi le groupe constitué de C₁₋₆-alkyle, C₃₋₆-cycloakyl-, C₃₋₆-cycloalkyl-C₁₋₃-alkyle, C₁₋₆-alkyl-CO-, C₃₋₆-cycloakyl-CO-, C₃₋₆-cycloakyl-C₁₋₃-alkyl-CO-, C₁₋₆-alkyl-SO₂-, C₃₋₆-cycloakyl-SO₂-, C₃₋₆-cycloakyl-C₁₋₃-alkyl-SO₂-, phényl-CO- et phényl-SO₂-,
R^{d} représente -O-Me
A représente -CH₂-CH₂-
éventuellement sous la forme de leurs tautomères, leurs racémiques, leurs énantiomères, leurs diastéréomères et leurs mélanges, ainsi qu'éventuellement de leurs sels d'addition d'acide inoffensifs sur le plan pharmacologique.

2. Composés selon la revendication 1, **caractérisés en ce que**
R^{b} et R^{c} identiques ou différents, représentent hydrogène ou C₁₋₃-alkyle
éventuellement sous la forme de leurs tautomères, leurs racémiques, leurs énantiomères, leurs diastéréomères et leurs mélanges, ainsi que, éventuellement, de leurs sels d'addition d'acide inoffensifs sur le plan pharmacologique.

3. Composés de formule (I) selon l'une des revendications 1 à 2, destinés à être utilisés comme médicaments.

4. Composés de formule (I) selon la revendication 3, destinés à être utilisés dans le traitement des maladies inflammatoires ou allergiques des voies respiratoires.

5. Composés de formule (I) selon la revendication 3, destinés à être utilisés dans le traitement d'une maladie sélectionnée parmi le groupe constitué de la bronchite chronique, la bronchite aiguë, la bronchite liée à une infection virale ou bactérienne ou à des champignons ou des helminthes, la bronchite allergique, la bronchite toxique, la bronchopneumopathie chronique obstructive (BPCO), l'asthme (intrinsèque ou allergique), l'asthme pédiatrique, les bronchectasies, l'alvéolite allergique, la rhinite allergique ou non-allergique, la sinusite chronique, la fibrose kystique ou la mucoviscidose, le déficit en alpha-1-antitrypsine, la toux, l'emphysème pulmonaire, les pneumopathies interstitielles, l'alvéolite, l'hyperréactivité des voies respiratoires, les polypes nasaux, les oedèmes pulmonaires, la pneumopathie de diverses origines, telle qu'induite par les rayons ou par aspiration ou infectieuse, les collagénoses telles que le lupus érythémateux, la sclérodermie généralisée, la sarcoïdose et M. Boeck.

6. Composés de formule (I) selon la revendication 3, destinés à être utilisés dans le traitement d'états de maladies inflammatoires ou allergiques impliquant des réactions auto-immunes.

7. Composés de formule (I) selon la revendication 3, destinés à être utilisés dans le traitement d'une maladie sous forme de tumeurs bégnines ou malignes.

8. Formulation pharmaceutique contenant un composé de formule (I) selon l'une des revendications 1 ou 2.

9. Formulation pharmaceutique applicable oralement selon la revendication 8, contenant un composé de formule (I) selon la revendication 1 ou 2.

10. Combinaisons médicamenteuses qui, outre un ou plusieurs composés de formule (I) selon l'une des revendications 1 ou 2, contiennent comme autre principe actif un ou plusieurs composés qui sont sélectionnés parmi les classes des bêtamimétiques, anticholinergiques, corticostéroïdes, autres inhibiteurs de PDE4, antagonistes de LTD4, inhibiteurs de l'EGFR, agonistes de la dopamine, antihistaminiques H1, antagonistes du PAF et inhibiteurs de la PI3-kinase ou doubles ou triples combinaisons de ceux-ci.
